# EUROPEAN PATENT APPLICATION

(11) **EP 4 372 095 A1**
(43) Date of publication of application: **22.05.2024**
(21) Application number: 22841973.5
(22) Date of filing: 30.06.2022
(51) Int. Cl.: C12P 19/34, C12N 1/21, C12N 15/31, C12N 15/52, C12N 15/57

(54) **METHOD FOR PRODUCING PLASMID DNA USING ESCHERICHIA COLI**

(30) Priority: 16.07.2021 JP 2021117545
(71) Applicant: Kaneka Corporation, Osaka-shi, Osaka 530-8288 (JP); Kaneka Eurogentec S.A., 4102 Seraing (BE); Université Catholique de Louvain, 1348 Louvain-la-Neuve (BE)
(72) Inventor: TOKUNAGA Tomohisa, Takasago-shi, Hyogo 676-8688 (JP); NISHIYAMA Tozo, Takasago-shi, Hyogo 676-8688 (JP); MINAKUCHI Kazunobu, Takasago-shi, Hyogo 676-8688 (JP); CHO Seung Hyun, 1150 Bruxelles (BE); COLLET Jean-Francois, 1435 Hevillers (BE); DEGHELT Michael, 1300 Schaerbeek (BE)
(74) Representative: Hoffmann Eitle
(86) International application number: PCT/JP2022/026275
(87) International publication number: WO 2023/286629

(57) **Abstract**

Plasmid DNA can be efficiently recovered from bacterial cells when producing plasmid DNA using *E. coli.* Provided is a method for producing plasmid DNA including the following steps (a) to (c): (a) a step of preparing *E. coli,* which has a mutation in a gene region associated with maintaining outer membrane properties and has a desired plasmid; (b) a step of culturing *E. coli* of (a); and (c) a step of recovering a desired plasmid from bacterial cells after culture.

## Description

### Technical Field

The present invention relates to a method for producing plasmid DNA using *Escherichia coli.*

### Background Art

Plasmid DNA is used as a principal agent in gene therapy and DNA vaccines and also plays an important role in a wide range of applications, including as a viral vector for gene therapy, a virus vaccine, and a raw material for mRNA production.

As a method for producing plasmid DNA, a fermentation method is known, in which a transformant obtained by allowing an *E. coli* host strain to carry a desired plasmid is cultured, and plasmid DNA is extracted from the cultured *E. coli* strain. Non-Patent Literature 1 discloses an alkaline lysis method as a method for extracting *E. coli* plasmid DNA. Specifically, the alkaline lysis method is a method in which the membrane structure of *E. coli* is disrupted (dissolved) with an alkali, and plasmid DNA in the cytoplasm is extracted outside the bacterial cells. The extracted plasmid DNA is usually highly purified by membrane treatment, chromatography, or the like. This alkaline lysis method has been particularly widely used as a method for extracting plasmid DNA. However, it is known that in this method, once the mixing ratio of the bacterial cells and the alkaline solution deviates from the appropriate range, dissolution becomes insufficient, and the recovery rate decreases (Non-Patent Literature 2). Therefore, for example, in order to treat a large amount of bacterial cells on an industrial production scale, in addition to accurate control of the mixing ratio, a large amount of reagents is required.

Patent Literature 1 discloses a method for mass production of pharmaceutical-grade plasmid DNA by eluting plasmid DNA from cells without using enzymes, organic solvents, and mutagenic reagents and purifying the plasmid DNA by fractional PEG precipitation and chromatography.

### Citation List

### Patent Literature

Patent Literature 1: JP H09-509313 A (1997)

### Non-Patent Literature

Non-Patent Literature 1: H. C. Bimnoim and J. Doly, Nucleic Acids Res. Vol. 7, No.6, pp. 1513-1523 (1979)
Non-Patent Literature 2: QIAGEN (registered trademark) Plasmid Purification: Protocol and Troubleshooting 06/2005

### Summary of Invention

### Technical Problem

As mentioned above, in the conventional production of plasmid DNA using *E. coli,* especially on an industrial production scale, the efficiency of bacterial cell lysis in the extraction step from bacterial cells (e.g., alkali-SDS treatment using the alkaline lysis method) is not necessarily high. This was one of the causes of a decrease in the recovery rate of plasmid DNA. Patent Literature 1 discloses a method for mass-producing pharmaceutical-grade plasmid DNA but does not particularly disclose a method for increasing the efficiency of cell lysis per alkaline solution.

An object of the present invention is to provide a method for producing plasmid DNA using *E. coli,* whereby objective plasmid DNA can be efficiently recovered from bacterial cells.

### Solution to Problem

The present inventors have conducted concentrated studies, and, as a result, discovered that the use of the *E. coli* strain having a mutation of a gene associated with maintaining outer membrane properties would enhance extraction efficiency, that is, improving the DNA recovery rate by increasing the rate of recovering plasmid DNA from bacterial cells and/or expanding the amount of plasmid produced per se. This has led to the completion of the present invention.
(1) A method for producing plasmid DNA, comprising the following steps (a) to (c):
   (a) a step of preparing *E. coli,* which has a mutation in a gene region associated with maintaining outer membrane properties and has a desired plasmid;
   (b) a step of culturing the *E. coli* of (a); and
   (c) a step of recovering a desired plasmid from bacterial cells after culture.
(2) The method according to (1), wherein the gene region associated with maintaining outer membrane properties is a gene region associated with maintaining physical and/or mechanical outer membrane properties.
(3) The method according to (2), wherein the gene region associated with maintaining physical and/or mechanical outer membrane properties is a gene region directly associated with maintaining physical and/or mechanical outer membrane properties.
(4) The method according to (3), wherein the gene region directly associated with maintaining physical and/or mechanical outer membrane properties is at least one selected from the group consisting of sequences encoding structural proteins of *pal, lpp, ompA, ybiS, ycfS, erfK, tolA, tolB, nlpI, bamD, bamA, lolA, lolB, lolC, lolD, lolE, lptE, lptD, lptA, IptB, IptF, lptG, slyB, mrcA, mrcB, lpoA, lpoB, tolQ,* and *tolR;* and nucleotide sequences encoding signal peptides thereof, promoter sequences, and SD sequences.
(5) The method according to any one of (1) to (4), wherein the *E. coli* has a mutation in an amino acid sequence of at least one peptide selected from the group consisting of Pal, Lpp, OmpA, YbiS, YcfS, ErfK, TolA, TolB, NlpI, BamD, BamA, LolA, LolB, LolC, LolD, LolE, LptE, LptD, LptA, LptB, LptF, LptG, SlyB, MrcA, MrcB, LpoA, LpoB, TolQ, and TolR; and signal peptides thereof.
(6) The method according to (5), wherein the *E. coli* has a mutation in an amino acid sequence of at least one peptide selected from the group consisting of Pal, Lpp, OmpA, YbiS, YcfS, ErfK, and signal peptides thereof.
(7) The method according to any one of (1) to (6), wherein the mutation is a complete disruption.
(8) The method according to any one of (1) to (6), wherein the mutation is a partial mutation.
(9) The method according to (8), wherein the mutation is a partial mutation of a signal peptide.
(10) The method according to (8), wherein the mutation is a partial mutation of a structural protein.
(11) The method according to (10), wherein the structural protein is a structural protein having a partial mutation of any of the following (A) to (E):
   (A) a structural protein binding to or contacting with any of an outer membrane, a peptidoglycan layer, or an inner membrane and thus having a substitution or disruption in an amino acid residue at the site of binding or contacting and/or in the vicinity thereof;
   (B) a structural protein having an enzymatic activity of allowing another protein to bind to a peptidoglycan layer and having a substitution or disruption in an amino acid residue at its active site and/or in the vicinity thereof;
   (C) a structural protein serving as an enzyme involved in forming a peptidoglycan layer and having a substitution or disruption in an amino acid residue at its active site and/or in the vicinity thereof;
   (D) a structural protein serving as a protein involved in outer membrane transport of a lipoprotein present in a periplasm fraction and having a substitution or deletion in an amino acid residue at a site binding to the lipoprotein and/or in the vicinity thereof; and
   (E) a structural protein serving as a protein involved in outer membrane transport of a lipopolysaccharide and having a substitution or deletion in an amino acid residue at a site binding to the lipoprotein and/or in the vicinity thereof.
(12) The method according to (11), wherein the structural protein is the structural protein (A), which is at least one selected from the group consisting of Pal, Lpp, and OmpA.
(13) The method according to (12), wherein the structural protein is Pal, which has the mutation in an N-terminal region thereof.
(14) The method according to (12), wherein the structural protein is Lpp, which has the mutation in a C-terminal region thereof.
(15) The method according to (12), wherein the structural protein is OmpA, which has the mutation in a C-terminal region thereof.
(16) The method according to (11), wherein the structural protein is the structural protein (B), which is at least one selected from the group consisting of YbiS, YcfS, and ErfK.
(17) The method according to any of (1) to (16), wherein the *E. coli* is derived from the B strain or the K12 strain.
(18) A method for preparing *E. coli* for plasmid DNA production, comprising the steps (i) and (ii):
   (i) a step of causing a mutation in a gene region associated with maintaining outer membrane properties of *E. coli*; and
   (ii) a step of preparing *E. coli,* which has the mutation obtained in (i) and has a desired plasmid.
(19) *E. coli* for producing plasmid DNA, which has a mutation in a gene region associated with maintaining outer membrane properties and has a desired plasmid.

This description includes part or all of the content as disclosed in the description and/or drawings of Japanese Patent Application No. 2021-117545, which is a priority literature of the present application.

### Advantageous Effects of Invention

According to the present invention, a method for producing plasmid DNA using *E. coli,* whereby plasmid DNA can be efficiently recovered from bacterial cells, can be provided. Embodiments of the Invention

### 1. Method for Producing Plasmid DNA

The method for producing plasmid DNA according to the present invention comprises the following steps (a) to (c):
(a) a step of preparing *E. coli,* which has a mutation in a gene region associated with maintaining outer membrane properties and has a desired plasmid;
(b) a step of culturing the *E. coli* of (a); and
(c) a step of recovering a desired plasmid from bacterial cells after culture.

The method according to the present invention has the characteristics described above, and the method thus enables efficient recovery of objective plasmid DNA from bacterial cells of *E. coli.*

In producing pharmaceuticals and the like, it is required to produce large quantities at low industrial costs. The same applies to the production of plasmid DNA. To improve plasmid DNA production efficiency, the development of high-productivity hosts, high-productivity culture production methods, and high-yield purification methods is underway. The method for producing plasmid DNA according to the present invention (hereafter also referred to as "the method of the present invention") is a method for improving the plasmid extraction efficiency or increasing the amount of plasmid produced per bacterial cell by changing physical and/or mechanical outer membrane properties of *E. coli* as a host for producing plasmid DNA. The method enables reducing the cost of the extraction step by improving productivity by improving plasmid production efficiency per unit area of the facility and/or reducing the amount of buffer used. Specifically, reducing the amounts of materials with a reduced volume to be processed, reducing labor costs by shortening the extraction time, and the like can be realized.

### 1-1 E. coli Strain

An *E. coli* used in the method of the present invention is a genetically-modified strain, a parent *E. coli* strain thereof is not particularly limited, and any known *E. coli* strain can be used. In particular, the B strain, a strain derived from the B strain, the K12 strain, or a strain derived from the K12 strain can be preferably used. Alternatively, the hybrid strain of B strain and K12 strain, the HB101 strain, can be used. Examples of known strains derived from the B strain include the BL21 strain and the REL606 strain. Examples of known strains derived from the K12 strain include the W3110 strain, the DH10B strain, the BW25113 strain, the DH5α strain, the MG1655 strain, the JM109 strain, and the RV308 strain.

### 1-2 Gene Associated with Maintaining Outer Membrane Properties

The *E. coli* strain used in the method of the present invention is a gene mutant strain having a mutation in one or more gene regions associated with maintaining outer membrane properties (hereafter also referred to as "the gene associated with maintaining outer membrane properties").

It is known that the form of *E. coli* is influenced by the membrane structure composed of an outer membrane, a peptidoglycan (PG) layer, and an inner membrane, and the form of *E. coli* is reflected in the outer membrane properties. The main components of the outer membrane are phospholipid and lipopolysaccharide (LPS), the main component of the PG layer is a polymer consisting of a polysaccharide and a peptide, and the main component of the inner membrane is phospholipid. The membrane structure is composed of an outer membrane protein, an inner membrane protein, a protein linking the outer membrane and the PG layer, a protein linking the proteins described above, a protein linking the PG layer and the inner membrane, a protein linking the outer membrane-PG layer-inner membrane, and the like. The term "outer membrane properties" used herein refers to physical and/or mechanical outer membrane properties, which are maintained by various proteins present in the outer and inner membranes and by the PG layer and also maintained directly or indirectly by the functions of various proteins present in the outer and inner membranes.

The term "physical outer membrane properties" used herein refers to the form, size, membrane integrity, membrane fluidity, permeability, or the like of *E. coli* formed with the outer membrane. The term "mechanical outer membrane properties" used herein refers to the mechanical strength of the outer membrane and the surface condition, for example, the hardness or surface structure of *E. coli.* These properties are related to the growth and division *of E. coli,* the maintenance of bacterial cell form, and the sensitivity and responsiveness to external factors such as temperature, osmotic pressure, and substances.

Proteins, which are directly associated with maintaining physical and/or mechanical outer membrane properties and present in the inner membrane or periplasm space, include proteins that bind to, come into contact with, or penetrate the outer membrane, the PG layer, and/or the inner membrane and/or proteins that interact with these proteins so as to be involved in maintaining outer membrane properties as well as an enzyme that links these proteins to the PG layer and an enzyme involved in PG layer formation.

Pal is a lipoprotein that exists abundantly in the periplasm between the outer membrane and the inner membrane in Gram-negative bacteria. The N-terminal side is anchored to the outer membrane, and the C-terminal region interacts with the PG layer such that Pal contributes to maintaining outer membrane properties.

Lpp is a lipoprotein that exists most abundantly in the periplasm between the outer membrane and the inner membrane in Gram-negative bacteria. The N-terminal side is anchored to the outer membrane, and lysine on the C-terminal side and the PG layer are covalently bonded. Lpp contributes to maintaining outer membrane properties by linking the outer membrane and PG and keeping the space distance therebetween.

OmpA is an outer membrane protein that exists most abundantly in the periplasm between the outer membrane and the inner membrane in Gram-negative bacteria and plays an important role in maintaining outer membrane properties. OmpA specifically interacts with the PG layer, particularly residues R256 and D241 directly interacting with PG.

The binding between Lpp and the PG layer is catalyzed by LD-transpeptidase (Ldt), which includes ErfK (LdtA), YbiS (LdtB), and YcfS (LdtC). Ldt catalyzes the covalent bond between Lpp and PG, thereby crosslinking the outer membrane and PG and contributing to outer membrane structure maintenance.

In addition to the above, NlpI, BamD, BamA, LolA, LolB, LptE, LptD, LptA, LpoA, LpoB, and SlyB are inserted in the outer membrane or the inner membrane or present between the outer membrane and the inner membrane so as to be associated with maintaining physical and/or mechanical outer membrane properties.

Tol proteins, including TolA, TolB, TolQ, and TolR, constitute the Tol-Pal system with Pal, play the role of a bridge between the inner membrane, the outer membrane, and PG, and contribute to maintaining outer membrane properties.

The Lol mechanism is involved in inserting an outer membrane protein, such as Lpp or Pal, into the outer membrane and is composed of LolA/B/C/D/E.

The Lpt mechanism is important for LPS transport from the inner membrane to the outer layer of the outer membrane and is associated with maintaining physical and/or mechanical outer membrane properties. This Lpt mechanism is composed of LptA/B/C/D/E/F/G.

The Bam mechanism is involved in inserting an outer membrane protein such as OmpA into the outer membrane. The proteins involved in the mechanism include BamA/B/C/D/E, which form a complex.

MrcA, MrcB, LpoA, and LpoB are involved in PG layer formation and associated with maintaining physical and/or mechanical outer membrane properties.

As a result of concentrated studies, the present inventors have discovered that a mutation in the gene region of each functional protein associated with maintaining physical and/or mechanical outer membrane properties described above would enhance plasmid DNA production efficiency by either or both of the broadly divided following mechanisms: improving the extraction efficiency and expanding the amount of plasmid produced per se. For example, although gene regions involved in each mechanism are listed below, this does not necessarily indicate that the above-described functional classification of gene products is involved in only one mechanism, and mutations are not limited to the mutations in gene regions exemplified below.
(Mechanism 1) Enhancement of Extraction Efficiency and Expansion of Amount of Plasmid Produced: *pal, lpp, ompA, ybiS, ycfS, erfK, tolB, nlpI, bamD, bamA, lolA, lolB, lolC, lolD, lolE, IptE, IptD, lptB, lptF,* and *lptG*
(Mechanism 2) Expansion of Amount of Plasmid Produced: *skyB, tolA,* and *tolR*

The *E. coli* gene mutant strain used in the method of the present invention may have mutations in two or more gene regions associated with maintaining physical and/or mechanical outer membrane properties. In this case, a mutation may be included in two or more gene regions belonging to one group selected from gene region groups associated with the two mechanisms described above, or a mutation may be included in two or more gene regions, including at least one gene region of each group. Examples of an *E. coli* gene mutant strain having a mutation in two or more gene regions include *E. coli* gene mutant strains having a mutation in gene regions, such as *ompA*+*Lpp, ompA*+*ybiS*+*ycsF*+*erfK, ybiS*+*ycsF*+*erfK, ompA+ybiS, ompA+ycsF, ompA+erfK, ompA+ybiS+ycsF, ompA*+*ybiS*+*erfK, ompA+ycsF+erfK, pal+ompA, pal+lpp, pal+ybis+ycsF+erfK, pal+ybiS, pal+ycsF, pal*+*erJK, pal+ybiS+ycsF, pal+ybiS+erfK,* and *pal*+*ycsF*+*erfK.*

Meanwhile, examples of a protein indirectly associated with maintaining physical and/or mechanical outer membrane properties include: (1) chaperone proteins present in the periplasm space for controlling the folding of the outer membrane protein (such as *SurA* (e.g., GenBank: CAD6022301.1), *FkpA* (e.g., GenBank: CAD6001217.1), *Spy* (e.g., GenBank: CAD6012704.1), *Skp* (e.g., GenBank: CAD6017209.1), *DegP* (e.g., GenBank: CAD6021993.1), *UgpB* (e.g., GenBank: CAD5993943.1), and *OsmY*(e.g*.,* GenBank: EGT67405.1)); (2) catalyst proteins for controlling the folding of the outer membrane protein (such as *DsbA* (e.g., NCBI Reference: NP_418297.1), *DsbC* (e.g., NCBI Reference: NP_417369.1), and *DsbD* (e.g., NCBI Reference: NP_418559.1)); (3) enzymes involved in the formation and decomposition of the PG layer; and (4) proteins associated with extracellular stress response (such as *Rcs* (e.g., GenBank: PSY27771.1), *Cpx* (e.g., NCBI Reference: NP_312809.1), and *SigmaE* (e.g., GenBank: BAE76749.1)).

More specifically, the phrase "gene associated with maintaining outer membrane properties" used herein refers to a gene and gene region associated with maintaining physical and/or mechanical outer membrane properties of *E. coli.* Further specifically, the phrase "gene maintaining outer membrane properties" used herein refers to an *E. coli* gene, that is, a nucleic acid contained in *E. coli* (DNA or RNA, preferably DNA) involved in the expression of a protein associated with maintaining physical and/or mechanical outer membrane properties of *E. coli.* The gene associated with maintaining outer membrane properties is not particularly limited and may be any gene region associated with maintaining outer membrane properties regardless of whether directly or indirectly; however, it is preferably a gene region directly associated with the same. It is preferable to define the gene associated with maintaining outer membrane properties as at least one gene region selected from the group consisting of, for example, *pal, lpp, ompA, ybiS, ycfS, erfK, tolA, tolB, nlpI, bamD, bamA, lolA, lolB, lolC, lolD, lolE, lptE, lptD, IptA, lptB, lptF, IptG, slyB, mrcA, mrcB, lpoA, lpoB, tolQ,* and *tolR.* The gene region *pal, lpp, ompA, ybiS, ycfS,* or *erfK* is particularly preferable, although not limited thereto. More specifically, a gene region comprising a nucleotide sequence having 60% or higher, 70% or higher, 80% or higher, 85% or higher, 90% or higher, 95% or higher, 96% or higher, 97% or higher, 98% or higher, 99% or higher, or 100% sequence identity to the nucleotide sequence of any one of SEQ ID NOS: 1 to 30, 54 to 59, and 62 to 83 is preferable.

The gene associated with maintaining outer membrane properties comprises Small RNA and non-coding RNA for controlling the gene expression described above. These mutations attenuate the expression of the gene associated with maintaining outer membrane properties described above.

The "sequence identity" of nucleotide sequences can be determined herein by a method, sequence analysis software, or the like well-known in the art. Examples include the blastn program of the BLAST algorithm and the fasta program of the FASTA algorithm. In the present invention, the "sequence identity" of a nucleotide sequence to be evaluated to the nucleotide sequence X is a value determined by aligning the nucleotide sequence X and the nucleotide sequence to be evaluated, introducing gaps, according to need, so as to maximize the extent of nucleotide consistency therebetween, and determining a frequency (%) of identical nucleotides appearing at the same sites in the nucleotide sequences including the gaps.

The term "signal peptide" used herein refers to a peptide sequence of approximately 15 to 30 amino acids present on the N-terminal side of a target protein, the peptide directing transport and localization of the protein. The signal peptide is usually cleaved with a signal peptidase after transport and localization. The term "structural protein" used herein refers to a protein after cleavage of the signal peptide, which corresponds to the structure of the body of the protein.

Among the genes associated with maintaining outer membrane properties described above, *pal* is a gene to express the outer membrane lipoprotein Pal. SEQ ID NO: 1 shows an example of the nucleotide sequence of a structural gene encoding Pal *of E. coli.* SEQ ID NO: 2 shows the nucleotide sequence (signal nucleotide sequence) encoding a *pal* signal peptide.

*lpp* is a gene to express the major outer membrane lipoprotein Lpp. SEQ ID NO: 3 shows an example of the nucleotide sequence of a structural gene encoding Lpp of *E. coli* SEQ ID NO: 4 shows the nucleotide sequence encoding a Lpp signal peptide.

*ompA* is a gene to express the outer membrane protein OmpA. SEQ ID NO: 5 shows an example of the nucleotide sequence of a structural gene encoding OmpA *of E. coli.* SEQ ID NO: 6 shows the nucleotide sequence encoding an OmpA signal peptide.

*ybiS* is a gene to express the periplasm protein YbiS. YbiS is known as a transpeptidase that binds Lpp and PG. SEQ ID NO: 54 shows an example of the nucleotide sequence of a structural gene encoding YbiS of *E. coli.* SEQ ID NO: 55 shows the nucleotide sequence encoding a YbiS signal peptide.

*ycfS* is a gene to express YcfS, which is a paralog of YbiS. SEQ ID NO: 56 shows an example of the nucleotide sequence of a structural gene encoding YcfS *of E. coli.* SEQ ID NO: 57 shows the nucleotide sequence encoding a YcfS signal peptide.

*erfK* is a gene to express ErfK, which is a paralog of YbiS. SEQ ID NO: 58 shows an example of the nucleotide sequence of a structural gene encoding ErfK *of E. coli.* SEQ ID NO: 59 shows the nucleotide sequence encoding an ErfK signal peptide.

*tolA* is a gene to express one of the proteins constituting the Tol/Pal system, TolA. The Tol/Pal system is a complex of a plurality of proteins existing in Gram-negative bacteria, and it is reported to be associated with outer membrane invagination during cell division and maintenance of membrane structure. SEQ ID NO: 7 shows an example of a nucleotide sequence of a structural gene encoding TolA of *E. coli.*

*tolB* is a gene to express one of the proteins constituting the Tol/Pal system, TolB. SEQ ID NO: 8 shows an example of the nucleotide sequence of a structural gene encoding TolB of *E. coli.* SEQ ID NO: 9 shows the nucleotide sequence encoding a TolB signal peptide.

*mepS* is a gene to express peptidoglycan DD-endopeptidase/peptidoglycan LD-peptidase, MepS. SEQ ID NO: 10 shows an example of the nucleotide sequence of a structural gene encoding MepS of *E. coli.* SEQ ID NO: 11 shows the nucleotide sequence encoding a MepS signal peptide.

*nlpI* is a gene to express a lipoprotein, NlpI. SEQ ID NO: 12 shows an example of the nucleotide sequence of a structural gene encoding NlpI of *E. coli.* SEQ ID NO: 13 shows the nucleotide sequence encoding a NlpI signal peptide.

*bamD* is a gene to express an outer membrane protein, BamD. SEQ ID NO: 15 shows an example of the nucleotide sequence of a structural gene encoding BamD of *E. coli.* SEQ ID NO: 16 shows the nucleotide sequence encoding a BamD signal peptide.

*dppA* is a gene to express a dipeptide binding protein, DppA. SEQ ID NO: 19 shows an example of the nucleotide sequence of a structural gene encoding DppA *of E. coli.* SEQ ID NO: 20 shows the nucleotide sequence encoding a DppA signal peptide.

*ecnB* is a gene to express a toxin lipoprotein, EcnB. SEQ ID NO: 21 shows an example of the nucleotide sequence of a structural gene encoding EcnB of *E. coli.* SEQ ID NO: 22 shows the nucleotide sequence encoding an EcnB signal peptide.

*mrcA* is a gene to express peptidoglycan glycosyl transferase/peptidoglycan DD-transpeptidase, MrcA. SEQ ID NO: 23 shows an example of the nucleotide sequence of a structural gene encoding MrcA of *E. coli.*

*mrcb* is a gene to express peptidoglycan glycosyl transferase/peptidoglycan DD-transpeptidase, MrcB. SEQ ID NO: 24 shows an example of the nucleotide sequence of a structural gene encoding MrcB of *E. coli.*

*oppA* is a gene to express a periplasmic oligopeptide-binding protein, OppA. SEQ ID NO: 25 shows an example of a nucleotide sequence of a structural gene encoding OppA of *E. coli.* SEQ ID NO: 26 shows the nucleotide sequence encoding an OppA signal peptide.

*slyB* is a gene to express peptidyl-prolyl cis-trans isomerase, SlyB. SEQ ID NO: 27 shows an example of a nucleotide sequence of a structural gene encoding SlyB of *E. coli.* SEQ ID NO: 28 shows the nucleotide sequence encoding a SlyB signal peptide.

*tolQ* is a gene to express one of the proteins constituting the Tol/Pal system, TolQ. SEQ ID NO: 29 shows an example of the nucleotide sequence of a structural gene encoding TolQ of *E. coli.*

*tolR* is a gene to express one of the proteins constituting the Tol/Pal system, TolR. SEQ ID NO: 30 shows an example of the nucleotide sequence of a structural gene encoding TolR of *E. coli.*

*bamA* is a gene to express an outer membrane protein, BamA. SEQ ID NO: 62 shows an example of the nucleotide sequence of a structural gene encoding BamA of *E. coli.* SEQ ID NO: 63 shows the nucleotide sequence encoding a BamA signal peptide.

*lolA* is a gene to express a protein involved in lipoprotein transport from the inner membrane to the outer membrane, LolA. SEQ ID NO: 64 shows an example of the nucleotide sequence of a structural gene encoding LolA of *E. coli.* SEQ ID NO: 65 shows the nucleotide sequence encoding a LolA signal peptide.

*lolB* is a gene to express an outer membrane protein involved in lipoprotein transport, LolB. SEQ ID NO: 66 shows an example of the nucleotide sequence of a structural gene encoding LolB of *E. coli.* SEQ ID NO: 67 shows the nucleotide sequence encoding a LolB signal peptide.

*lolC* is a gene to express an inner membrane protein involved in lipoprotein transport, LolC. SEQ ID NO: 68 shows an example of the nucleotide sequence of a structural gene encoding LolC of *E. coli.*

*lolD* is a gene to express an ATP-binding protein involved in lipoprotein transport, LolD. SEQ ID NO: 69 shows an example of the nucleotide sequence of a structural gene encoding LolD of *E. coli.*

*lolE* is a gene to express an inner membrane protein involved in lipoprotein transport, LolE. SEQ ID NO: 70 shows an example of the nucleotide sequence of a structural gene encoding LolE of *E. coli.*

*IptE* is a gene to express a lipoprotein involved in LPS transport, LptE. SEQ ID NO: 71 shows an example of the nucleotide sequence of a structural gene encoding LptE of *E. coli.* SEQ ID NO: 72 shows the nucleotide sequence encoding an LptE signal peptide.

*IptD* is a gene to express an outer membrane protein involved in LPS transport, LptD. SEQ ID NO: 73 shows an example of the nucleotide sequence of a structural gene encoding LptD of *E. coli.* SEQ ID NO: 74 shows the nucleotide sequence encoding an LptD signal peptide.

*IptA* is a gene to express a protein involved in LPS transport, LptA. SEQ ID NO: 75 shows an example of the nucleotide sequence of a structural gene encoding LptA of *E. coli.* SEQ ID NO: 76 shows the nucleotide sequence encoding an LptA signal peptide.

*IptB* is a gene to express an ATP-binding protein involved in LPS transport, LptB. SEQ ID NO: 77 shows an example of the nucleotide sequence of a structural gene encoding LptB of *E. coli.*

*IptF* is a gene to express a protein involved in LPS transport, LptF. SEQ ID NO: 78 shows an example of the nucleotide sequence of a structural gene encoding LptF of *E. coli.*

*IptG* is a gene to express a protein involved in LPS transport, LptG. SEQ ID NO: 79 shows an example of the nucleotide sequence of a structural gene encoding LptG of *E. coli.*

*lpoA* is a gene to express an outer membrane protein involved in PG synthesis, LpoA. SEQ ID NO: 80 shows an example of the nucleotide sequence of a structural gene encoding LpoA of *E. coli.* SEQ ID NO: 81 shows the nucleotide sequence encoding an LpoA signal peptide.

*lpoB* is a gene to express an outer membrane protein involved in PG synthesis, LpoB. SEQ ID NO: 82 shows an example of the nucleotide sequence of a structural gene encoding LpoB of E. coli. SEQ ID NO: 83 shows the nucleotide sequence encoding an LpoB signal peptide.

In addition to the genes associated with maintaining outer membrane properties described above, *E. coli* having mutations in the *arcA* and/or *cyoA* gene regions may also be used. *arcA* is a gene to express ArcA (aerobic respiration control). SEQ ID NO: 14 shows an example of the nucleotide sequence of a structural gene encoding ArcA of *E. coli. cyoA* is a gene to express a membrane protein, CyoA, constituting the respiratory system. SEQ ID NO: 17 shows an example of a nucleotide sequence of a structural gene encoding CyoA of *E. coli.* SEQ ID NO: 18 shows the nucleotide sequence encoding a CyoA signal peptide.

### 1-3. E. coli Strain Having Mutation in Gene Associated with Maintaining Outer Membrane Properties

The term "mutation" of a gene refers to a state in which an alteration in a nucleotide sequence is added to a gene existing in nature (such gene is referred to as a "wild-type" gene). The "mutation" is not particularly limited, provided that a certain kind of alteration is added to the nucleotide sequence of the original gene. It may be either complete disruption or partial mutation. The term "partial mutation" used herein refers to a mutation other than a complete disruption of the relevant gene region and refers explicitly to an insertion, substitution, or partial disruption. A mutation in the present invention is preferably a partial mutation and particularly preferably a partial disruption or substitution. When the genetic mutation is a partial disruption or substitution, it is possible to attenuate the stability of the membrane structure compared to the wild strain and maintain the growth ability of *E. coli,* making it possible to mass culture *E. coli* in ajar fermenter or the like.

The *E. coli* gene mutant strain used in the method of the present invention has genetic mutation that alters expression of one or more genes associated with maintaining outer membrane properties. The term "genetic mutation that alters expression of one or more genes associated with maintaining outer membrane properties" used herein refers to genetic mutation that significantly alters activity of a protein encoded by the gene, in comparison with activity of the parent strain. In particular, genetic mutation that attenuates activity of a protein encoded by the gene, compared with activity of the parent strain, is preferable, and such mutation encompasses genetic mutation that would completely quench the activity. When protein activity is attenuated, the expression levels of mRNA, which is a transcription product of the target gene, or a protein, which is a translation product thereof, are lowered, or mRNA, which is a transcription product of the target gene, or a protein, which is a translation product thereof, would not normally function as mRNA or a protein.

The term "disruption" of a gene used herein refers to deletion or damage, with "deletion" being preferable. Examples of the partial mutation include disruption, insertion, and substitution, with partial disruption or partial substitution being preferable. The term "partial disruption" refers to disruption of a part of a gene or protein. The term "partial substitution" refers to substitution of a part of a nucleotide sequence of a gene or an amino acid sequence of a protein with another sequence, that is, substitution of one or more amino acids, which also includes amino acid substitution due to missense mutation caused by substitution of a single nucleotide. Hereafter, "one or more genes associated with maintaining outer membrane properties " to be mutated may also be simply referred to as a "target gene." A target gene may be either a gene associated with maintaining outer membrane properties or two or more genes associated with maintaining outer membrane properties. Alternatively, mutations may be present at a plurality of sites of a gene associated with maintaining outer membrane properties.

When a part of the coding region of the amino acid sequence of the protein encoded by the target gene is to be deleted or substituted as the partial deletion or substitution, any region, such as the N-terminal region, the internal region, the C-terminal region, or other regions may be deleted. When the gene is deleted, it also encompasses that the reading frames of the upstream and downstream sequences of the region to be deleted are inconsistent. It is possible to delete or substitute at least a part of the protein encoded by the target gene, such as a region consisting of the number of amino acids that is, for example, at least one amino acid or at least 10%, at least 20%, at least 30%, at least 40%, at least 50%, at least 60%, at least 70%, at least 80%, at least 90%, or at least 95% of the total number of the amino acids. Alternatively, 100% of the region can be deleted (complete disruption). For example, an *E. coli* gene mutant strain lacking at least a region from the start codon to the stop codon of the target gene of genomic DNA may be used.

According to the present invention, as the partial disruption or substitution, mutation of a region binding to or contacting with the inside of the coding region of the amino acid sequence of the protein encoded by the target gene, the outer membrane, the PG layer, or the inner membrane, and/or a region of binding or contact between proteins, and/or the enzymatic activity center, the catalyst region, and/or any amino acid residue in the vicinity thereof is effective.

According to the present invention, the *E. coli* mutation may be in the coding region or the expression control sequence of the structural protein. When the coding region of the structural protein has the mutation, which particularly results in a partial mutation of the structural protein, the structural protein is preferably a structural protein having any partial mutation from (A) to (E) below:
(A) a structural protein binding to or contacting with any of an outer membrane, a peptidoglycan layer, or an inner membrane and thus having a substitution or disruption in an amino acid residue at the site of binding or contacting and/or in the vicinity thereof;
(B) a structural protein having an enzymatic activity of allowing a different protein to bind to a peptidoglycan layer and having a substitution or disruption in an amino acid residue at its active site and/or in the vicinity thereof;
(C) a structural protein serving as an enzyme involved in forming a peptidoglycan layer and having a substitution or disruption in an amino acid residue at its active site and/or in the vicinity thereof;
(D) a structural protein serving as a protein involved in outer membrane transport of a lipoprotein present in a periplasm fraction and having a substitution or deletion in an amino acid residue at a site binding to the lipoprotein and/or in the vicinity thereof; and
(E) a structural protein serving as a protein involved in outer membrane transport of a lipopolysaccharide and having a substitution or deletion in an amino acid residue at a site binding to the lipoprotein and/or in the vicinity thereof.

When the structural protein having a partial mutation is the structural protein (A), it is preferably at least one selected from the group consisting of Pal, Lpp, and Omp. In the case of Pal, the mutation thereof is preferably in the N-terminal region, including a signal peptide. In the case of Lpp, the mutation thereof is preferably in the C-terminal region. In the case of OmpA, the mutation thereof is preferably in the C-terminal region.

When the structural protein having a partial mutation is the structural protein (B), it is preferably at least one selected from the group consisting of YbiS, YcfS, and ErfK.

The term "expression control sequence" used herein is not particularly limited, provided that such nucleotide sequence can be associated with expression of the coding region, and the expression control sequence may be a nucleotide sequence encoding a signal peptide, a promoter sequence, and/or a Shine-Dalgarno (SD) sequence (e.g., 5'-AGGAGGA-3'). The *E*. *coli* gene mutant strain of the present invention encompasses a mutant strain with complete disruption or partial mutation of a nucleotide sequence encoding a signal peptide, a promoter sequence, and/or an SD sequence.

Other examples of mutation of the target gene of genomic DNA in the *E. coli* strain include introduction of missense mutation, introduction of a stop codon (nonsense mutation), and introduction of frameshift mutation via addition or deletion of 1 or 2 nucleotides into or from the amino acid sequence coding region of the gene in genomic DNA.

Mutation of the target gene of genomic DNA in the *E. coli* strain can be also achieved by, for example, substitution or insertion of other gene sequence into the expression control sequence or the amino acid sequence coding region of the gene in genomic DNA. The other sequence may be inserted into any region of the gene. It also encompasses that reading frames of upstream and downstream sequences of the site of insertion are inconsistent. While the "other gene sequence" is not particularly limited, an example thereof is a marker gene. Examples of marker genes include, but are not limited to, drug-resistant markers reacting with drugs, such as kanamycin, ampicillin, tetracycline, and chloramphenicol, and auxotrophic markers reacting with nutrients, such as leucine, histidine, lysine, methionine, arginine, tryptophan, and uracil.

The *E. coli* gene mutant strain can be obtained by, for example, mutagenesis, gene recombination, gene expression control using RNAi, gene editing, or the like.

Mutagenesis can be performed via ultraviolet irradiation or via treatment using a common agent causing mutation, such as N-methyl-N'-nitro-N-nitrosoguanidine (MNNG), ethyl methanesulfonate (EMS), methyl methanesulfonate (MMS), or the like.

Gene recombination can be performed in accordance with a known technique, for example, the technique described in FEMS Microbiology Letters 165 (1998) 335-340, JOURNAL OF BACTERIOLOGY, Dec. 1995, pp. 7171-7177, Curr Genet 1986;10(8):573-578, or WO 98/14600, gene editing such as CRISPR-Cas9, synthetic biology, or the like.

A method in which a gene associated with maintaining outer membrane properties of *E. coli* having a mutation is prepared and integrated into the genome of an arbitrary *E. coli* strain can be preferably employed. Examples of methods for preparing a gene associated with maintaining outer membrane properties of *E. coli* having a mutation include methods known to a person skilled in the art, such as overlap PCR and total synthesis using adequate oligonucleotide primers. The prepared gene associated with maintaining outer membrane properties of *E. coli* having a partial mutation can be adequately integrated into a host cell in accordance with an adequately known method. Examples of methods include a method involving the use of the Red-recombinase system (Datsenko K.A. and Wanner B.L., 2000, Proc. Natl. Acad. Sci., U.S.A., 97 (12), 6640-6645) and a method based on the homologous recombination mechanism of a host cell (Link A. J. et al., 1997, J. Bacteriol., 179, 6228-6237). In particular, the method described in Link et al. by which a foreign gene other than the gene of the *E. coli* genome would not be integrated into the genome is preferable.

Examples of methods for preparing recombinant vectors of the gene associated with maintaining outer membrane properties having a partial mutation include, but are not particularly limited to, ligation, In-fusion (Clontec), PCR, and total synthesis. When an *E. coli* host is used, for example, a recombinant vector can be introduced into the host cell by the calcium chloride method, electroporation, or other methods, although the methods are not limited thereto.

Mutation of a target gene in genomic DNA of an *E. coli* strain can be realized by substitution of the gene in genomic DNA of the *E. coli* strain with a deletion gene or a marker gene. The term "deletion gene" used herein refers to an inactive gene modified so as not to produce a protein that normally functions by deleting a part or the whole of the target gene or substituting a part of the target gene. Examples of deletion genes include a linear DNA comprising an arbitrary sequence without functions of the target gene wherein the linear DNA comprises upstream and downstream sequences of the target sites of substitution (i.e., a part or the whole of the target gene) on genomic DNA at the both ends of the arbitrary sequence or wherein the linear DNA comprises upstream and downstream sequences of the target sites of substitution on genomic DNA which are directly connected to each other. An example of a marker gene is a linear DNA comprising a sequence of the marker gene and upstream and downstream sequences of the target sites of substitution (i.e., a part or the whole of the target gene) on genomic DNA at both ends of the marker sequence. The *E. coli* strain may be transformed with the linear DNA to cause homologous recombination in regions upstream and downstream of the target site of substitution in genomic DNA of the host strain. Thus, the target site of substitution can be substituted with the sequence of the linear DNA in a single step. As a result of such substitution, the target gene can be deleted from genomic DNA of the *E. coli* strain.

A marker gene may be removed after the substitution, according to need. When a marker gene is to be removed, sequences for homologous recombination or flippase recognition target (FRT) sequences may be added to both ends of the marker gene, so as to efficiently remove the marker gene.

When the *E. coli* gene mutant strain is a strain in which the *pal* gene has a mutation, examples thereof include an *E. coli* strain with mutation of a gene (SEQ ID NO: 1) encoding Pal (comprising the amino acid sequence as shown in SEQ ID NO: 31) in the *E. coli* genome and an *E. coli* strain with mutation of an expression control sequence, for example, an *E. coli* strain with mutation of a gene (SEQ ID NO: 2) encoding a signal peptide of Pal (SEQ ID NO: 32). Examples of mutation of the signal peptide include a substitution of another signal peptide and a substitution of another signal peptide having a mutation. Examples of such mutation include a mutation in which a hydrophobic amino acid in the hydrophobic region of a signal peptide is substituted with a hydrophilic amino acid, preferably a mutation in which leucine at position 14 is substituted with asparagine.

Pal mutation may be complete disruption or partial mutation. Partial mutation of a sequence other than a signal nucleotide sequence is preferably partial disruption, although not particularly limited. Examples of *E. coli* strains comprising a gene encoding Pal with partial disruption include an *E. coli* strain with partial disruption of a gene (SEQ ID NO: 1) encoding Pal (comprising the amino acid sequence as shown in SEQ ID NO: 31) in the *E. coli* genome and an *E. coli* strain with partial disruption of a gene (SEQ ID NO: 2) encoding a signal peptide (SEQ ID NO: 32) of Pal. The site to be partially disrupted from Pal may be the site described in, for example, Cascales E. and Lloubes R., 2004, Mol. Microbiol., 51 (3), 873-885.

A strain with partial mutation of Pal can be prepared by introducing a gene comprising a gene encoding Pal with partial mutation in a Pal-deficient strain lacking a Pal-encoding gene to express Pal with partial mutation. In the present invention, an *E. coli* strain with partial mutation of Pal (SEQ ID NO: 30) is preferably used, and the partial mutation is more preferably partial disruption.

The *E. coli* strain with partial mutation of Pal is not particularly limited; however, the mutation is preferably a substitution or disruption of a site where Pal binds to or contacts with the outer membrane, the PG layer, and/or another protein associated with maintaining outer membrane properties and/or any amino acid residue in the vicinity thereof. Examples thereof include an *E. coli* strain comprising, in the genome, a gene (SEQ ID NO: 34) encoding Pal (SEQ ID NO: 33) lacking amino acids 3 to 17, an *E. coli* strain comprising, in the genome, a gene (SEQ ID NO: 36) encoding Pal (SEQ ID NO: 35) lacking amino acids 19 to 43, an *E. coli* strain comprising, in the genome, a gene (SEQ ID NO: 38) encoding Pal (SEQ ID NO: 37) lacking amino acids 44 to 62, an *E. coli* strain comprising, in the genome, a gene (SEQ ID NO: 40) encoding Pal (SEQ ID NO: 39) lacking amino acids 62 to 93, an *E. coli* strain comprising, in the genome, a gene (SEQ ID NO: 42) encoding Pal (SEQ ID NO: 41) lacking amino acids 94 to 121, an *E. coli* strain comprising, in the genome, a gene (SEQ ID NO: 44) encoding Pal (SEQ ID NO: 43) lacking amino acids 123 to 152, an *E. coli* strain comprising, in the genome, a gene (SEQ ID NO: 46) encoding Pal (SEQ ID NO: 45) lacking amino acids 126 to 129, and an *E. coli* strain comprising, in the genome, a gene (SEQ ID NO: 48) encoding Pal (SEQ ID NO: 47) lacking amino acids 144 to 147.

Pal with the partial mutation may comprise an amino acid sequence derived from the amino acid sequence as shown in any of SEQ ID NO: 33, 35, 37, 39, 41, 43, 45, or 47 by substitution, deletion, and/or addition of one or a plurality of amino acids. The term "one or a plurality of' refers to, for example, 1 to 80, preferably 1 to 70, preferably 1 to 60, preferably 1 to 50, preferably 1 to 40, preferably 1 to 30, preferably 1 to 20, preferably 1 to 15, preferably 1 to 10, preferably 1 to 5, preferably 1 to 4, preferably 1 to 3, preferably 1 to 2, or preferably 1.

Pal with the partial mutation may comprise an amino acid sequence having 60% or higher sequence identity to the amino acid sequence as shown in any of SEQ ID NO: 33, 35, 37, 39, 41, 43, 45, or 47. The "sequence identity" may be 70% or higher, 80% or higher, 85% or higher, 90% or higher, 95% or higher, 96% or higher, 97% or higher, 98% or higher, 99% or higher, or 100%.

The sequence identity of amino acid sequences can be determined herein by a method, sequence analysis software, or the like well-known in the art. Examples include the blastp program of the BLAST algorithm and the fasta program of the FASTA algorithm. In the present invention, the "sequence identity" of an amino acid sequence to be evaluated to the amino acid sequence X is a value determined by aligning the amino acid sequence X and the amino acid sequence to be evaluated, introducing gaps, according to need, so as to maximize the extent of amino acid consistency therebetween, and determining a frequency (%) of identical amino acids appearing at the same sites in the amino acid sequences including the gaps.

Examples *of E. coli* gene mutant strains having a mutation of the *lpp* gene include an E. *coli* strain with mutation of a gene (e.g., the nucleotide sequence of SEQ ID NO: 3) encoding Lpp (e.g., Lpp comprising the amino acid sequence of SEQ ID NO: 49) in the *E. coli* genome and an *E. coli* strain with mutation of a gene (e.g., the nucleotide sequence as shown in SEQ ID NO: 4) encoding an expression control sequence, for example, a signal peptide of *Lpp* (e.g., a signal peptide having the amino acid sequence of SEQ ID NO: 60). Examples of the mutation mode for the *Lpp* amino acid sequence of SEQ ID NO: 49 include, but are not particularly limited to, a mutation in the C-terminal region of Lpp, specifically a mutation of disrupting lysine at position 58 of *Lpp* or substituting arginine at position 57 of *Lpp* with an uncharged amino acid, preferably leucine. Alternative examples thereof include, but are not limited to, a mutation predicted to partially disrupt the α-helical structure of *Lpp*, for example, in the *Lpp* amino acid sequence of SEQ ID NO: 49, a mutation in which amino acid SSD at positions 11 to 13 of *Lpp* (positions 31 to 33 including the signal peptide) is substituted with VNFS. In the case of mutation of the signal peptide, examples of the mutation also include a mutation in which glycine at position 14 of the Lpp signal peptide is substituted with aspartic acid, a substitution of another signal peptide, and a substitution of another signal peptide having a mutation.

Examples *of E. coli* gene mutant strains having a mutation of the *ompA* gene include an *E. coli* strain with mutation of a gene (e.g., the nucleotide sequence of SEQ ID NO: 5) encoding OmpA (e.g., OmpA comprising the amino acid sequence of SEQ ID NO: 50) in the *E. coli* genome and an *E. coli* strain with mutation of a gene (e.g., the nucleotide sequence as shown in SEQ ID NO: 6) encoding an expression control sequence, for example, a signal peptide of OmpA (e.g., a signal peptide having the amino acid sequence of SEQ ID NO: 61). Examples of the mutation mode for the amino acid sequence of SEQ ID NO: 50 include, but are not particularly limited to, a mutation in the C-terminal region of OmpA, specifically a mutation in which arginine at position 256 is substituted with an uncharged or negatively-charged amino acid, preferably glutamic acid or alanine and a mutation in which aspartic acid at position 241 is substituted with an uncharged or positively-charged amino acid, preferably asparagine. Examples thereof also include, but are not limited to, a mutation in which the C-terminal region of OmpA is disrupted, for example, a region ranging from glutamic acid at position 241 or glutamic acid at position 256 or from an amino acid located upstream thereof to the C-terminus is disrupted. In the case of mutation of the signal peptide, examples of the mutation also include a mutation in which the amino acid of the OmpA signal peptide, as shown in SEQ ID NO: 61, is substituted with another amino acid, a substitution of another signal peptide, and a substitution of another signal peptide having a mutation.

When the *E. coli* gene mutant strain is a strain having a mutation in the *ybiS, ycfS,* and *erfK* genes, examples of thereof include *E. coli* with mutation of the genes encoding YbiS, YcfS, and ErfK in the E. coli genome and E. coli with mutation of the genes encoding various signal peptides. Examples of the mutation mode for various amino acid sequences include, but are not particularly limited to, a mutation in which one, two, or three of the above-described three genes is disrupted. A mutation of the expression control sequence is also included. In the case of mutation of the signal peptide, examples of the mutation include a mutation in which amino acids in various signal peptides are substituted with different amino acids, a substitution of another signal peptide, and a substitution of another signal peptide having a mutation.

When the *E. coli* gene mutant strain is a strain with mutation of the *tolA, tolB, tolQ,* and *tolR* genes involved in the Tol-*Pal* system, examples thereof include *E. coli* with mutation of the genes encoding TolA, TolB, TolQ, and TolR in the *E. coli* genome and a gene encoding an expression control sequence, for example, a signal peptide. The mutation mode for the amino acid sequences of various Tol proteins is not particularly limited. Examples thereof also include a mutation in which an amino acid in the signal peptide is substituted with another amino acid, a substitution of another signal peptide, and a substitution of another signal peptide having a mutation.

When the *E. coli* gene mutant strain is a strain with mutation of the *lolA, lolB, lolC, lolD,* and *lolE* genes involved in the Lol mechanism, examples thereof include *E. coli* with mutation of the genes encoding LolA, LolB, LolC, LolD, and LolE in the *E. coli* genome and a gene encoding an expression control sequence, for example, a signal peptide. The mutation mode for the amino acid sequences of various Lol proteins is not particularly limited. Examples thereof also include a mutation in which an amino acid in the signal peptide is substituted with another amino acid, a substitution of another signal peptide, and a substitution of another signal peptide having a mutation.

When the *E. coli* gene mutant strain is a strain with mutation of the *IptE, lptD, lptA, IptB, IptF,* and *IptG* genes involved in the Lpt mechanism, examples thereof include *E. coli* with mutation of the genes encoding LptE, LptD, LptA, LptB, LptF, and LptG in the *E. coli* genome and a gene encoding an expression control sequence, for example, a signal peptide. The mutation mode for the amino acid sequences of various Lpt proteins is not particularly limited. One example is *Lpt*DΔ330-352 with partial disruption of 330th to 352nd amino acids of the amino acid sequence of LptD (M. Braun and T. J. Silhavy (2002), Mol Microbiol., 45(5): 1289-302). Examples thereof also include a mutation in which an amino acid in the signal peptide is substituted with another amino acid, a substitution of another signal peptide, and a substitution of another signal peptide having a mutation.

When the *E. coli* gene mutant strain is a strain with mutation of the *bamD* and *bama* genes involved in the Bam mechanism, examples thereof include *E. coli* with mutation of the genes encoding BamD and BamA in the *E. coli* genome and *E. coli* with mutation of a gene encoding an expression control sequence, for example, a signal peptide. The mutation mode for the amino acid sequences of various Bam proteins is not particularly limited. One example is the mutant strain *BamA*101 with a reduced BamA expression level, in which EZ-Tn5 is inserted at a position 23 bp-upstream of the start codon of BamA. In the case of mutation of the signal peptide, examples of the mutation also include a mutation in which the amino acids in various signal peptides are substituted with different amino acids, a substitution of another signal peptide, and a substitution of another signal peptide having a mutation.

In addition to the above, when the *E. coli* gene mutant strain is a strain having a mutation in the *nlpl, slyB, mrcA, mrcB, lpoA,* or *lpoB* gene, examples of thereof include *E. coli* with mutation of the gene encoding NlpI, SlyB, MrcA, MrcB, LpoA, or LpoB and *E. coli* with mutation of the gene encoding a signal peptide. It is possible to delete or substitute a region consisting of at least one amino acid or at least 10%, at least 20%, at least 30%, at least 40%, at least 50%, at least 60%, at least 70%, at least 80%, at least 90%, or at least 95% of the total number of the amino acids in the target protein. Alternatively, 100% of the region may be deleted, although not particularly limited.

### 1-4. Step of Preparing Plasmid-having E. coli (Step (a))

The method of the present invention comprises:
(a) a step of preparing *E. coli,* which has a mutation in a gene region associated with maintaining outer membrane properties and has a desired plasmid.

### 1-4-1. Preparation of Plasmid

In step (a), *E. coli* "having a mutation in a gene region associated with maintaining outer membrane properties" to be a host can be prepared by the method described above. Step (a) further comprises allowing the *E. coli* gene mutant strain to have a plasmid.

As used herein, the terms "plasmid" and "plasmid DNA" refer to DNA molecules that exist outside the nucleus of *E. coli* and are inherited by daughter cells through cell division. In the present invention, it particularly refers to a DNA molecule artificially constructed outside a bacterial cell. Any known plasmid can be used. Examples of the plasmid that can be used include, but are not limited to, pBluescript vector, pET vector, pETduet vector, pGBM vector, pBAD vector, and pUC vector. Plasmids are often used in the form of circular duplexes.

Preferred examples of a plasmid borne by *E. coli* by the method of the present invention (i.e., plasmid DNA that is replicated and produced) include, but are not particularly limited to, those that can be used for gene therapy, DNA vaccines, and the like for humans or animals Such plasmid DNA preferably contains DNA molecules helpful in preventing or treating diseases, pathological conditions, and the like. Examples thereof include plasmid DNA containing a nucleotide sequence useful as a DNA vaccine, plasmid DNA useful per se, and protein expression plasmid DNA for animal cells. Examples of DNA molecules helpful in preventing or treating diseases, pathological conditions, and the like mentioned herein include those encoding antisense oligonucleotides, aptamers, and the like.

The molecular weight of a plasmid used in the present invention is not particularly limited; however, those with a molecular weight of 1 to 200 kbp, particularly 2 to 150 kbp, can be used.

### 1-4-2 Gene Introduction

In the method of the present invention, the plasmid is introduced into the *E. coli* gene mutant strain. A method for introducing plasmid into an *E. coli* strain is not particularly limited, and an expression vector can be introduced into an *E. coli* strain by a method of gene introduction, such as electroporation, the calcium chloride method, the competent cell method, or the protoplast method.

As described above, the *E. coli* gene mutant strain having a desired plasmid is prepared in step (a). Since the plasmid is replicated as *E. coli* divides and is inherited by daughter cells, step (a) corresponds to breeding *E. coli* having new genetic information.

### 1-5. Culture Step (Step (b))

The method of the present invention comprises a step of culturing the *E. coli* gene mutant strain prepared in step (a) (hereafter also referred to as "step (b)").

The *E. coli* gene mutant strain can be cultured using a known culture system such as a flask or ajar fermenter. Culture may be carried out in a large-volume (e.g., 30-L to 10,000-L) culture tank. In addition, the *E. coli* gene mutant strain can be cultured in an adequate medium. Culture may be performed by any batch culture, fed-batch culture, or continuous culture. The medium may be either a synthetic or natural medium, provided that it comprises nutrients necessary for the growth of the *E. coli* gene mutant strain, such as carbon sources, nitrogen sources, inorganic salts, and vitamins.

Any carbon sources can be used, provided that carbon sources are assimilable by the *E*. *coli* gene mutant strain. Examples of carbon sources include saccharides, such as glucose and fructose, alcohols, such as ethanol and glycerol, and organic acids, such as acetic acid.

Examples of nitrogen sources include ammonia, ammonium salt such as ammonium sulfate, a nitrogen compound such as amine, and a natural nitrogen source such as peptone.

Examples of inorganic salts include potassium phosphate, magnesium sulfate, sodium chloride, ferrous sulfate, and potassium carbonate.

Examples of vitamins include biotin and thiamine. In addition, substances required by the *E. coli* gene mutant strain to grow (e.g., a required amino acid for an amino acid-requiring strain) can be added, according to need.

In the method of the present invention, a medium supplemented with peptone, yeast extract, and sodium chloride is preferably used. Peptone is preferably soybean peptone. A preferable example of a medium supplemented with peptone, yeast extract, and sodium chloride is 2 x YT medium.

In the method of the present invention, culture conditions are not particularly limited. For example, culture is preferably carried out via shake culture or agitation culture. Culture is performed at 20°C to 50°C, preferably at 25°C to 45°C, and more preferably at 30°C to 42°C. A culture period is 3 hours to 5 days, and preferably 5 hours to 3 days.

### 1-6. Step of Recovering Plasmid DNA (Step (c))

The method of the present invention comprises a step of recovering objective plasmid DNA from bacterial cells of *E. coli* cultured in step (b). As a method for recovering plasmid DNA from bacterial cells of *E. coli,* known methods such as a lysis method and a density gradient ultracentrifugation method, as well as commercially available purification kits (for example, QIAprep Spin Miniprep Kit (QIAGEN)) can be used. The lysis method is not particularly limited, but for example, the alkaline lysis method described in Non-Patent Literature 1 can be used.

The procedure of the alkaline lysis method described in Non-Patent Literature 1 is summarized as follows. Bacterial cells are collected from the bacterial cell culture solution by a known method, such as centrifugation or filtration. The collected bacterial cells are treated with a lysozyme solution and then dissolved in a strong alkaline solution containing a surfactant such as SDS. A highly concentrated sodium acetate solution is added to the bacterial cell lysate on ice to precipitate proteins, high molecular weight RNA, and chromosomal DNA, followed by centrifugation and collection of the supernatant. Cold ethanol is added to the collected supernatant to precipitate the plasmid DNA, centrifugation is performed, and the supernatant is removed. The precipitate is suspended in sodium acetate/Tris-HCl, two volumes of ethanol are added, the suspension is cooled to -20°C, and then the precipitate is collected by centrifugation. After suspending the precipitate in sterile water, it is purified using agarose gel electrophoresis. Note that the alkaline lysis method is not particularly limited to the method described in Non-Patent Literature 1. Materials and conditions can be optimized as appropriate depending on the purpose.

As the lysis method, a method, in which bacterial cells are lysed with an alkaline solution and a surfactant, and then genomic DNA, proteins, and polymeric RNA are coprecipitated together with the surfactant by neutralization and removed, is commonly used. The types of alkaline materials, surfactants, and the like used, and conditions such as concentration, temperature, pH, and the like are not limited. In the lysis method, lysis efficiency can be increased by weakening the membrane structure using lysozyme before lysis. Further, the purity of the plasmid can be increased by adding RNase to degrade RNA, which becomes an impurity. Furthermore, plasmid purity can also be increased by adding calcium chloride during neutralization so as to promote RNA precipitation.

The plasmid DNA purification method after bacterial cell lysis is not limited to the purification by agarose gel electrophoresis described in Non-Patent Literature 1. Any known purification technique, such as a dedicated column, may be used.

In a specific aspect, the *E. coli* gene mutant strain used in the method of the present invention is advantageous in that it is an *E. coli* gene mutant strain having proteins with attenuated functions, which are directly associated with maintaining physical and/or mechanical outer membrane properties, that is, proteins with attenuated functions due to suppression the expression levels of the proteins, mutation, or deletion, it allows plasmid DNA to be efficiently extracted outside bacterial cells, thereby making it possible to recover plasmid DNA efficiently. In particular, in the alkaline extraction method, sensitivity to the alkaline-SDS solution is increased due to attenuated outer membrane structural stability, and the bacteriolytic effect of the alkaline-SDS solution is improved compared to the wild strain. In another aspect, the *E. coli* gene mutant strain used in the method of the present invention is characterized in that the mutant strain having an increased amount of plasmid produced compared to the wild strain because of having a mutation in a protein directly associated with maintaining physical and/or mechanical outer membrane properties, and thus the amount of plasmid produced per bacterial cell is more significant than usual, thereby making it possible to recover plasmid DNA efficiently. The use of the *E. coli* gene mutant strains in the aspects above allows for reducing the space by reducing the volume to be treated, i.e., increasing the amount of bacterial cells that can be treated at once, reducing materials due to reduced volume to be treated, and/or improving extraction efficiency, thereby making it possible to achieve large amounts of plasmid DNA recovered.

### 2. Method for Preparing E. coli for Plasmid DNA Production

The method for preparing an *E. coli* strain used for producing the plasmid DNA of the present invention (hereafter also referred to as "the method for preparing an *E. coli* strain of the present invention") comprises the following steps (i) and (ii):
(i) a step of causing a mutation in a gene region associated with maintaining outer membrane properties of *E. coli;* and
(ii) a step of preparing *E. coli,* which has the mutation obtained in (i) and has a desired plasmid.

The *E. coli* produced by the method for preparing *E. coli* of the present invention is advantageous in that due to the attenuation of physical or mechanical outer membrane properties, sensitivity to external stresses such as heat, a plasmid extraction reagent, and the like has increased, and thus, the bacteriolytic effect is improved compared to the wild strain, thereby making it possible to recover plasmid DNA efficiently. Alternatively, the *E. coli* produced by the method for preparing *E. coli* of the present invention is advantageous in that the amount of plasmid produced per bacterial cell per se increases, thereby making it possible to recover plasmid DNA efficiently.

### 2-1. Step of Genetic Mutation of E. coli (Step (i))

The method for preparing *E. coli* of the present invention comprises a step of mutating a gene associated with maintaining outer membrane properties of *E. coli* (hereafter also referred to as "step (i)"). The *E. coli* strains used in the method for preparing *E. coli* of the present invention are described in the section "1-1. *E. coli* Strain" of"1. Method for Producing Plasmid DNA" above. In addition, the genes associated with maintaining outer membrane properties subjected to mutation are described in the section "1-2. Gene Associated with Maintaining Outer Membrane Properties" above. Further, specific methods, conditions, and the like for modifying a gene associated with maintaining outer membrane properties of *E. coli* are described in the section "1-3. *E. coli* strain Having Mutation in Gene Associated with Maintaining Outer Membrane Properties" above.

### 2-2. Step of Preparing Plasmid-having E. coli (Step (ii))

The method for preparing *E. coli* of the present invention comprises a step of inserting a desired plasmid into the *E. coli* gene mutant strain obtained in step (i) so as to prepare plasmid-having *E. coli* (hereafter also referred to as "step (ii)"). The plasmid, the plasmid introduction method, and the like used in step (ii) are described in the section "1-4. Step of Preparing Plasmid-having *E. coli* (Step (a))" above.

### 3. E. coli for Plasmid DNA Production

The *E. coli* for plasmid DNA production of the present invention (hereafter also referred to as *"E. coli* of the present invention") is characterized by having a mutation in a gene region associated with maintaining outer membrane properties and having a desired plasmid. The *E*. *coli* of the present invention is advantageous in that the plasmid can be efficiently recovered during plasmid purification after culture.

Details of mutation of gene regions associated with maintaining outer membrane properties of the *E. coli* of the present invention are described in the section "1-2. Gene Associated with Maintaining Outer Membrane Properties" of"1. Method for Producing Plasmid DNA." Details of the structure and the like of plasmid DNA borne by the *E. coli* of the present invention are described in the section "1-4. Step of Preparing Plasmid-having *E. coli* (Step (a))" above.

### 4. Plasmid DNA

The present invention provides plasmid DNA produced using *E. coli.* Specifically, plasmid DNA produced according to "1. Method for Producing Plasmid DNA" is provided.

### Examples

The present invention is described in greater detail with reference to the following examples, although the present invention is not limited to these examples.

Genetic recombination techniques employed in the present invention, such as the polymerase chain reaction (PCR), gene synthesis, isolation and purification of DNA, treatment with a restriction enzyme, cloning of modified DNA, and transformation, are known to a person skilled in the art. The following examples were performed in accordance with the procedures described in the manufacturers' instructions for reagents and apparatuses unless otherwise specified.

In the following examples, PCR was carried out using Prime STAR HS DNA Polymerase (Takara Bio Inc.). The PCR product and the restriction enzyme reaction solution were purified using the QIAquick Gel Extraction Kit (QIAGEN). When a DNA fragment was to be prepared by a technique other than PCR, a common technique for gene synthesis was employed.

### [Example 1] Evaluation of Pal Mutant Strain of E. coli W3110 Strain

### (Example 1-1) Preparation of Pal Mutant Strain

A partially Pal-deficient strain of the *E. coli* W3110 strain was prepared with reference to the method described in Link A. J. et al. (J. Bacteriol., 1997, 179, 6228-6237). Various DNA fragments containing Pal-encoding nucleotide sequences with a disrupted region listed in Table 1 and having homology arms necessary for homologous recombination on the genome of approximately 200 bp upstream and downstream outside the *pal* gene were obtained by performing overlap PCR. This DNA fragment has an EcoRI restriction enzyme recognition sequence at the 5' end and a Sail restriction enzyme recognition sequence at the 3' end.

The prepared DNA fragments and cloning vectors were cleaved restriction enzymes EcoRI and Sail. Ligation was performed using each cleaved DNA fragment. The mutagenesis plasmid thus prepared was transformed into the W3110 strain by the calcium chloride method, and the mutagenesis-plasmid-having strain was selected using a tetracycline-containing medium. The genetic recombination operations to obtain the Pal mutant strain of interest were carried out based on the method described in Link A. J. et al.

**[Table 1]**

| Strain name | Mutation mode | SEQ ID NO. |
|---|---|---|
| Pa1Δ19-43 | Δ19-43 | 52 |
| Pa1Δ144-147 | Δ144-147 | 53 |

### (Example 1-2) Culture of Plasmid-having Strain

The gWiz (trademark) plasmid (Genlantis) was introduced into the Pal mutant strain by the electroporation method. A gWiz (trademark) plasmid-having strain was selected in a kanamycin-containing medium (50 mg/L).

Kanamycin was added to 3 mL of LB liquid medium (10 g/L triptone (Life Technologies Corporation), 5 g/L yeast extract (Dickinson and Company), 10 g/L sodium chloride (special grade, NACALAI TESQUE, INC.)) to result in a final concentration of 50 mg/L. A preculture liquid was added to result in an OD of 0.01. Culture was performed at 37°C and 300 rpm for 16 hours. The amount of bacterial cells in the culture solution was quantitatively determined using the OD600 value.

### (Example 1-3) Influence on Amount of Extraction Reagent Used on Amount of Plasmid Extracted

The QIAprep Spin Miniprep Kit (QIAGEN) was used for plasmid extraction. Bacterial cells were collected from 1 mL of the culture solution obtained in Example 1-2 by centrifugation. The thus obtained pellet was used. In this evaluation, the extraction reagents, P1, P2, and N3 buffers, were used under two conditions: a condition in which plasmid was extracted using the volume specified in the protocol (x1 condition); and a condition in which plasmid was extracted at one-fourth (1/4) the volume (x1/4 condition). Other operations followed the protocol. The obtained plasmid eluate was quantitatively determined using NanoDrop (trademark). In addition, the yield of plasmid DNA to bacterial cells (µg/mL/OD) was calculated from the OD and plasmid DNA concentration.

Table 2 shows the concentration of extracted plasmid DNA (µg/m L) and the yield of plasmid DNA to bacterial cells under each condition. When comparing the concentration of plasmid DNA extracted under the x1/4 condition with the concentration of plasmid DNA extracted under the x1 condition set to 100%. The concentration was 84% in WT, indicating that the extraction efficiency decreased by reducing the amount of extraction reagent. Meanwhile, the concentration was 96% for the Pa1Δ19-43 strain and 94% for the Pa1Δ144-147 strain, showing that the decrease in extraction efficiency due to the reduction in the amount of extraction reagent was suppressed compared to WT. This indicates that the Pal mutant strain is a strain from which plasmid can be easily extracted from the bacterial cells, and a high yield can be achieved even with the reduction in the amount of extraction reagent.

**[Table 2]**

| Host | Strain | OD | Plasmid DNA (µg/m L) | | Yield of plasmid DNA to bacterial cells (µg/mL/OD) | | Proportion (%) |
|---|---|---|---|---|---|---|---|
| | | | x1 | x1/4 | x1 | x1/4 | |
| W3110 | WT | 5.0 | 7.7 | 6.5 | 1.5 | 1.3 | 84 |
| | Pa1Δ19-43 | 5.4 | 7.1 | 6.8 | 1.3 | 1.2 | 96 |
| | Pa1Δ144-147 | 5.5 | 8.8 | 8.3 | 1.6 | 1.5 | 94 |

### (Example 1-4) Influence on Amount of Treated Bacterial Cells on Amount of Plasmid Extracted

The QIAprep Spin Miniprep Kit (QIAGEN) was used for plasmid extraction as in Example 1-3. Two conditions were examined: a condition in which bacterial cells obtained from 1 mL of the culture solution were treated (1-mL condition); and a condition in which bacterial cells obtained from 4 mL of the culture solution were treated (4-mL condition).

Table 3 shows the concentration of extracted plasmid DNA (µg/mL) and the yield of plasmid DNA to bacterial cells under each condition. When comparing the concentration of plasmid DNA extracted under the 4-mL condition with the concentration of plasmid DNA extracted under the 1-mL condition set to 100%. The concentration was 59% in WT, indicating that the extraction efficiency significantly decreased due to the increased amount of treated bacterial cells. Meanwhile, the concentration was 77% for the Pa1Δ19-43 strain and 81% for the Pa1Δ144-147 strain, showing that the decrease in extraction efficiency due to the increased amount of treated bacterial cells was suppressed compared to WT. It was also shown that under the 4-mL condition, more plasmid DNA could be extracted from the Pal mutant strain than from WT. This indicates that the Pal mutant strain is a strain from which plasmid can be easily extracted from the bacterial cells, and many bacterial cells can be treated in one extraction step.

**[Table 3]**

| Host | Strain | OD | Plasmid DNA (µg/mL) | | Yield of plasmid DNA to bacterial cells (µg/mL/OD) | | Proportion (%) |
|---|---|---|---|---|---|---|---|
| | | | 1mL | 4 mL | 1mL | 4 mL | |
| W3110 | WT | 5.1 | 6.6 | 3.9 | 1.3 | 0.8 | 59 |
| | Pa1Δ19-43 | 5.0 | 6.5 | 5.0 | 1.3 | 1.0 | 77 |
| | Pa1Δ144-147 | 5.6 | 8.9 | 7.2 | 1.6 | 1.3 | 81 |

### [Example 2] Evaluation of OmpA+Lpp, OmpA, and Lpp Mutant Strains of E. coli DH10B Strain

The OmpA+Lpp mutant strain (OmpA R256E+Lpp ΔK58), the OmpA mutant strain (OmpA R256E), and the Lpp mutant strain (Lpp ΔK58) of the *E. coli* DH10B strain were prepared by dsDNA recombination using the Lambda-Red system (Thomason et al. Plasmid. 2007 Sep;58(2):148-58). Specifically, the following two-step recombination method was used. First, *cat-sacB* was integrated into the target site and selected using chloramphenicol. Next, the *cat-sacB* cassette and the mutant gene locus were exchanged using the Lambda-Red method without changing the surrounding DNA region. For OmpA, *ompA*::*cat-sacB* (PCR product) was first integrated using the Lambda-Red method, and then recombination was performed with *ompA*::*ompAR256E.* For Lpp, *Ipp*::*cat-sacB* was first integrated, and then recombination was performed with *lpp::lpp*ΔK58*.*

For each mutant strain and the DH10 strain without mutation (WT), gWiz (trademark) plasmid (Genlantis) was introduced into each mutant strain by electroporation, and plasmid-having strains were selected using a kanamycin-containing medium (25 mg/L) as in Example 1.

Each bacterial strain was cultured in LB medium. Bacterial cells were collected by centrifuging 1 mL of the culture solution, and 150 µL of P1 (50 mM Tris-HCl, 80 mM EDTA, 100 µg/mL RNase A, pH 8.0) was added on ice. Next, 150 µL of P2 (100 mM NaOH, 1% SDS) was added and mixed by inverting several times. After incubation for 5 minutes at room temperature, 300 µL of P3 (3M potassium acetate, pH 8.0) was added on ice. After standing still on ice for 10 minutes, centrifugation was performed at 4°C and 12,500 rpm for 5 minutes. The supernatant was collected, mixed with 0.7 volumes of isopropanol, and centrifuged at 4°C and 12,500 rpm for 5 minutes. The supernatant was removed, and 300 µL of 70% ethanol was added, followed by centrifugation at 4°C and 12,500 rpm for 5 minutes. The supernatant was removed, dried, and resuspended in 20 µL of water.

Table 6 shows the absorbance of the culture solution, the amount of bacterial cells at the time of bacterial cell collection, and the amount of recovered DNA for each bacterial strain. Compared to WT, the amount of plasmid DNA produced was improved with the mutant strains OmpA_R256E+Lpp_ΔK58, OmpA_R256E, and Lpp_ΔK58.

**[Table 4]**

| Strain | OD | Amount of bacterial cells (g) | Amount of DNA recovered (ng/µL) |
|---|---|---|---|
| WT | 2.17±0.50 | 0.48±0.02 | 470±117 |
| OmpA R256E + lpp ΔK58 | 1.43±0.40 | 0.41±0.06 | 781±66 |
| OmpA R256E | 1.83±0.58 | 0.43±0.02 | 670±114 |
| Lpp ΔK58 | 2.23±0.59 | 0.45±0.04 | 600±126 |

### [Example 3] Evaluation of Pal and Lpp Mutant Strains of E. coli DH10B+recA Strain and OmpA Mutant Strain of E. coli DH10B Strain

A DHlOB+*recA* strain in which the *recA* gene was integrated into *E. coli* DH10B was created by the following method. First, a DNA fragment encoding *recA* was prepared. This DNA fragment was designed to have AflII restriction enzyme recognition sequences at both ends. The prepared DNA fragment and cloning vector were digested with restriction enzyme AflII. After restriction enzyme treatment, the cloning vector was dephosphorylated with alkaline phosphatase. The mutagenesis plasmid prepared by ligation using each cut DNA fragment was transformed into the DH10B strain by the calcium chloride method, and the mutagenesis-plasmid-having strain was selected using a tetracycline-containing medium. The DHlOB+recA strain was prepared as follows in the same manner as in Example 1.

A Pal mutant strain was prepared using the obtained DHlOB+*recA* strain in the same manner as in Example 1. A strain having the same disrupted region as Pa1Δ19-43 in Example 1-1 was prepared and designated as Pa1Δ19-43'. In addition, for Pal, various DNA fragments containing a nucleotide sequence encoding an amino acid sequence in which L at position 14 of the Pal signal peptide (SEQ ID NO: 31) was substituted with N and having homology arms necessary for homologous recombination on the genome of 300 bp upstream and downstream outside the *pal* gene were prepared. The Pal signal peptide point mutant strain (L14N) was prepared in the same manner as in Example 1. Further, for Lpp, DNA fragments containing a nucleotide sequence encoding an amino acid sequence in which G at position 14 of the Lpp signal peptide (SEQ ID NO: 60) was substituted with D and having homology arms necessary for homologous recombination on the genome of 500 bp upstream and downstream from the mutation point within the Lpp signal nucleotide sequence were prepared. The Lpp point mutant strain (G14D) was prepared in the same manner as in Example 1. Table 5 shows the mutation modes of the obtained Pal and Lpp mutant strains. It was confirmed that the Lpp mutant strain has, in addition to the above-described signal peptide point mutation, a mutation in which amino acid SSD at positions 11 to 13 of Lpp (positions 31 to 33 including the signal peptide) is substituted with VNFS. For the Omp mutant strain (R256E) of the *E. coli* DH10B strain, the mutant strain prepared in Example 2 was used as the OmpA mutant strain (R256E).

**[Table 5]**

| Strain name | Mutation mode |
|---|---|
| Pal L14N* | L14N* |
| Lpp G14D* | G14D*, S31_D33delinsVNFS* |

| | |
|---|---|
| *: Amino acid number containing a signal peptide | |

gWiz (trademark) plasmid (Genlantis) was introduced into each mutant strain by electroporation, and plasmid-having strains were selected using a kanamycin-containing medium (50 mg/L) as in Example 1. In addition to these mutant strains, plasmid introduction was performed similarly on a control, the DH10B+*recA* strain (hereafter referred to as "WT").

Each obtained plasmid-having strain was seeded on 3 mL LB medium (supplemented with 50µg/ mL kanamycin) and cultured at 37°C and 300 rpm for 8 hours. Next, the preculture solution was added to 40 mL of LB medium (supplemented with 50 µg/mL of kanamycin) and cultured at 37°C and 200 rpm for 16 hours.

The QIAprep Spin Miniprep Kit (QIAGEN) was used for plasmid extraction. Bacterial cells were collected by centrifuging 35 mL of the resulting culture solution. After measuring the whole cell weight (WCW) of the collected bacterial cells, the bacterial cells were suspended in the same weight of P1 buffer, thereby preparing a bacterial cell suspension. After that, plasmid extraction was performed according to the protocol for the extraction reagent, except that the amounts of bacterial cell suspension and each buffer described in the conditions of "x1" and "x4" in Table 4 were used. Table 4 shows the concentration of extracted plasmid DNA (mg/L) and the yield of plasmid DNA to bacterial cells under each condition.

**[Table 6]**

| Condition | Bacterial cell suspension (µL) | P1 (µL) | P2 (µL) | N1 (µL) |
|---|---|---|---|---|
| x1 | 20 | 230 | 250 | 350 |
| x4 | 80 | 170 | 250 | 350 |

**[Table 7]**

| Host | Strain | OD | Plasmid DNA (mg/L) | | Proportion (%) |
|---|---|---|---|---|---|
| | | | x1 | x4 | |
| DH10B+RecA | WT | 3.0 | 9.8±0.4 | 7.9±0.2 | 81 |
| | Pa1Δ19-43' | 3.2 | 11.1±0.9 | 10.1±0.8 | 91 |
| | Pal L14N* | 3.1 | 12.5±1.3 | 11.5±0.3 | 92 |
| | Lpp G14D* | 2.6 | 16.3±0.8 | 15.1±0.3 | 92 |
| DH10B | OmpA R256E | 2.3 | 13.4±0.1 | 12.1±0.2 | 91 |

| | | | | | |
|---|---|---|---|---|---|
| *: Amino acid number containing a signal peptide | | | | | |

In Pa1Δ19-43' and Pal L14N, the extraction efficiency was improved compared to WT and was equivalent to OmpA R256E. In addition, the extraction efficiency was also improved in Lpp G14D compared to WT and was equivalent to OmpA R256E. Moreover, a significant productivity improvement was confirmed. Favorable growth similar to that of WT was observed in the Pal and Lpp mutant strains.

### [Example 4] Evaluation of YbiS, YcsF, and ErfK Mutant Strains Using E. coli MG1665 Strain as Host

For the *E. coli* MG1665 strain, *ybiS::kan, ycsF::kan,* or *erfK::kan* from Keio Collection (Baba et al., Mol Syst Biol. 2006;2:2006.0008) was transduced into the MG1665 strain by P1 transduction (Curr Protoc Mol Biol. 2007 Jul; Chapter 1: Unit 1.17). The obtained mutant strain was selected using kanamycin. Then, the kanamycin-resistant gene was removed with FLP recombinase. P1 transduction and removal of the kanamycin-resistant gene were repeatedly performed using the thus obtained mutant strain as a host, thereby obtaining the Δ*ybiS*Δ*ycsF*Δ*erfK* strain. Mutagenesis of OmpA R256E of the OmpA *R256E*+Δ*ybiS*Δ*ycsF*Δ*erfK* strain was performed in the same manner as in Example 2. The OmpA R256E+LppΔK58 strain of the *E. coli* MG1665 strain was prepared in the same manner as in Example 2.

gWiz (trademark) plasmid (Genlantis) was introduced into each mutant strain by electroporation, and plasmid-having strains were selected using a kanamycin-containing medium (25 mg/L) as in Example 1. In addition to these mutant strains, plasmid introduction was performed similarly on a control, the MG1665 strain (hereafter referred to as "WT").

Each bacterial strain was cultured in an LB medium containing kanamycin, and the resulting culture solution was adjusted to have an OD of 1.0. Bacterial cells were collected by centrifuging 5 mL of the prepared solution. Extraction and column adsorption/washing operations were performed according to the protocol of the QIAquick Gel Extraction Kit. An elution buffer (10 mM Tris-HCl, pH 8.5) in an amount of 150 µL was applied to the column, left standing still at room temperature for 1 minute, and centrifuged. Next, the eluate was applied to the column again, allowed to stand still at room temperature for 1 minute, and centrifuged. Table 8 shows the ratio of the absorbance and the amount of recovered plasmid DNA for each bacterial strain when the value for WT is set to 1.

**[Table 8]**

| Host | Strain | OD/OD_{WT} | [DNA]/[DNA]_{WT} |
|---|---|---|---|
| MG1655 | WT | - | - |
| | ΔybiSΔycsFΔerfK | 0.97±0.03 | 2.69±0.52 |
| | OmpA R256E+ΔybiSΔycsFΔerfK | 0.24±0.06 | 5.15±1.11 |
| | OmpA R256E+LppΔK58 | 0.34±0.14 | 5.75±2.84 |

In the Δ*ybiS*Δ*ycsF*Δ*erfK* strain and the OmpA *R256E*+Δ*ybiS*Δ*ycsF*Δ*erfK* strain, the amount of plasmid DNA produced was improved compared to WT. In addition, in the OmpA *R256E*+Δ*ybiS*Δ*ycsF*Δ*erfK* strain, the amount of plasmid DNA produced was equivalent to that of the OmpA R256E+LppΔK58 strain.

### [Example 5] Evaluation of TolR and SlyB Mutant Strains Using E. coli BW25113 Strain as Host

The gWiz (trademark) plasmid (Genlantis) was introduced into mutant strains of *tolR::kan* (Δ*tolR*) and *slyB::kan* (Δ*slyB*) from Keio Collection (acquired from Horizon Discovery Ltd.) by the electroporation method using the *E. coli* BW25113 strain as a host. The gWiz plasmid used herein was gWiz-Ampr, in which the kanamycin-resistant gene was substituted with an ampicillin-resistant gene. The objective plasmid-having strain was selected using an ampicillin-containing medium. In addition to these mutant strains, plasmid introduction was performed similarly on a control, the BW25113 strain (hereafter referred to as "WT").

Each obtained plasmid-having strain was seeded on 3 mL LB medium (supplemented with 50µg/ mL ampicillin) and cultured at 37°C and 300 rpm for 8 hours. Next, the preculture solution was added to 40 mL of LB medium (supplemented with 50 µg/mL of ampicillin) and cultured at 37°C and 200 rpm for 16 hours.

The QIAprep Spin Miniprep Kit (QIAGEN) was used for plasmid extraction. Bacterial cells were collected by centrifuging 35 mL of the resulting culture solution. After measuring the whole cell weight (WCW) of the collected bacterial cells, the bacterial cells were suspended in the same weight of P1 buffer, thereby preparing a bacterial cell suspension. After that, plasmid extraction was performed according to the protocol for the extraction reagent, except that the amounts of bacterial cell suspension and each buffer described in the condition of "x1" in Table 9 were used. Table 10 shows the ratio of the absorbance and the amount of recovered plasmid DNA for each bacterial strain when the value for WT is set to 1.

**[Table 9]**

| Condition | Bacterial cell suspension (µL) | P1 (µL) | P2 (µL) | N3 (µL) |
|---|---|---|---|---|
| x1 | 25 | 100 | 125 | 175 |

**[Table 10]**

| Host | Strain | OD/OD_{WT} | [DNA]/[DNA]_{WT} |
|---|---|---|---|
| BW25113 | WT | - | - |
| | ΔtolR | 0.57 | 1.49 |
| | ΔslyB | 0.94 | 1.69 |

The amounts of plasmid produced in Δ*tolR* and Δ*slyB* were approximately 1.5 times and 1.7 times those of WT, respectively.

### [Example 6] Evaluation of TolB and TolQ Mutant Strains Using E. coli BW25113 Strain as Host

The same operations as in Example 5 were performed for obtaining mutant strains, introducing plasmids, and culturing. The QIAprep Spin Miniprep Kit (QIAGEN) was used for plasmid extraction. Bacterial cells were collected by centrifuging 35 mL of the resulting culture solution. After measuring the whole cell weight (WCW) of the collected bacterial cells, the bacterial cells were suspended in the same weight of P1 buffer, thereby preparing a bacterial cell suspension. After that, plasmid extraction was performed according to the protocol for the extraction reagent, except that the amounts of bacterial cell suspension and each buffer described in the conditions of "x1" and "x2" in Table 11 were used. Table 12 shows the extraction efficiency of the plasmid DNA (mg/L) extracted under each condition compared with the plasmid DNA recovered under the x1 condition with an extraction efficiency of 100%.

**[Table 11]**

| Condition | Bacterial cell suspension (µL) | P1 (µL) | P2 (µL) | N3 (µL) |
|---|---|---|---|---|
| x1 | 25 | 100 | 125 | 175 |
| x2 | 50 | 75 | 125 | 175 |

**[Table 12]**

| Host | Strain | OD | Plasmid DNA (mg/L) | | Proportion (%) (x2/x1)*100 |
|---|---|---|---|---|---|
| | | | x1 | x2 | |
| BW25113 | WT | 4.7 | 9.4±0.1 | 7.1±0.3 | 76 |
| | ΔtolB | 3.2 | 8.9±0.6 | 8.3±0.2 | 93 |
| | ΔtolQ | 3.1 | 8.9±0.6 | 7.6±0.4 | 85 |

When comparing plasmid DNA recovered under the x2 condition with the concentration of plasmid DNA extracted under the x1 condition set to 100%, WT showed an extraction efficiency of 76%. Meanwhile, the extraction efficiency was 93% and 85% in Δ*tolB* and Δ*tolQ,* respectively, and both improved compared to WT. This shows that the above-described mutant strain is a strain from which plasmid DNA can be easily recovered from the bacterial cells.

### [Example 7] Evaluation of Protein Mutant Strain Involved in Outer Membrane Structure Maintenance Using E. coli BW25113 Strain as Host

The same operations as in Example 6 were performed for obtaining mutant strains, introducing plasmid DNA, culturing, and extracting plasmid DNA. Table 13 shows the plasmid DNA (mg/L) extracted under each condition and the extraction efficiency when the plasmid DNA recovered under the x1 condition is set to 100%.

**[Table 13]**

| Host | Strain | OD | Plasmid DNA (mg/L) | | Proportion (%) (x2/x1)*100 |
|---|---|---|---|---|---|
| | | | x1 | x2 | |
| BW25113 | WT | 4.7 | 9.9±0.4 | 6.1±0.2 | 62 |
| | ΔmrcA | 4.9 | 10.8±0.3 | 8.3±0.6 | 77 |
| | ΔmrcB | 4.7 | 11.2±0.6 | 9.6±0.5 | 86 |
| | ΔnlpI | 1.5 | 1.1±0.1 | 1.2±0.1 | 109 |
| | ΔlpoA | 4.8 | 14.5±0.3 | 11.2±0.2 | 77 |
| | ΔlpoB | 4.7 | 11.7±0.3 | 8.5±0.5 | 73 |
| | ΔlolA | 4.7 | 11.7±0.4 | 8.6±0.4 | 74 |

When comparing plasmid DNA recovered under the x2 condition with plasmid DNA recovered under the x1 condition with an extraction efficiency of 100%, WT showed an extraction efficiency of 62%. Meanwhile, the extraction efficiency was higher than WT, with Δ*mrcA* at 77%, Δ*mrcB* at 86%, Δ*nlpI* at 109%, Δ*lpoA* at 77%, Δ*lpoB* at 73%, and Δ*lolA* at 74%. This shows that the above-described mutant strain is a strain from which plasmid DNA can be easily recovered from the bacterial cells. In addition, in all mutant strains except Δ*nlpI,* more plasmid DNA was recovered under the x1 condition than that for WT.

### [Example 8] Evaluation of Lpt Mutant Strain Using E. coli MC4100 Strain as Host

The LptDΔ330-352 mutant strain of the *E. coli* MC4100 strain (M. Braun and T. J. Silhavy, (2002), Mol Microbiol., 45(5): 1289-302) was acquired from the author. The same operations as in Example 5 were performed for introducing plasmids and culturing. In addition to these mutant strains, the same operations were performed similarly on a control, the MC4100 strain (hereafter referred to as "WT").

The obtained culture solution was prepared to have an OD of 5.0, 1 mL of the prepared solution was centrifuged to collect bacterial cells, and plasmid extraction was performed using the QIAprep Spin Miniprep Kit (QIAGEN) using the method in Example 1-3. Table 14 shows the extraction efficiency of the plasmid DNA (mg/L) extracted under each condition compared with the plasmid DNA recovered under the x1 condition with an extraction efficiency of 100%.

**[Table 14]**

| Host | Strain | OD | Plasmid DNA (ng/µL) | | Proportion (%) ((x1/4)/x1)*100 |
|---|---|---|---|---|---|
| | | | x1 | x1/4 | |
| MC4100 | WT | 1.2 | 146.0 | 114.2 | 78 |
| | LptDΔ330-352 | 1.0 | 128.4 | 121.8 | 95 |

When comparing plasmid DNA recovered under the x1/4 condition with plasmid DNA recovered under the x1 condition with an extraction efficiency of 100%, WT showed an extraction efficiency of 78%. Meanwhile, LptDΔ330-352 showed a higher extraction efficiency of 95% than WT. This shows that the above-described mutant strain is a strain from which plasmid DNA can be easily recovered from the bacterial cells.

### [Example 9] Evaluation of L,D-transpeptidase (Ldt) Mutant Strain and OmpA+Ldt Double Mutant Strain Using E. coli DH10B strain as Host

For the *E. coli* DH10B strain, mutant strains (Δ*erfK,* Δ*ybiS,* Δ*ycfS,* Δ*erfK*Δ*ybiS)* and double and multiple mutant strains of OmpA R256E and Ldt (OmpA R256E Δ*erfK, OmpA* R256E Δ*ybiS,* OmpA R256E Δ*ycfS,* OmpA R256E Δ*erfK*Δ*ybiS*) associated with L,D-transpeptidase were prepared by dsDNA recombination using the Lambda-Red system. The same operations as in Example 8 were performed for introducing plasmid, culturing, and extracting plasmid. In addition to these mutant strains, the same operations were performed similarly on a control, the DH10B strain (hereafter referred to as "WT"). Table 15 shows the extraction efficiency of the plasmid DNA (mg/L) extracted under each condition compared with the plasmid DNA recovered under the x1 condition with an extraction efficiency of 100%.

**[Table 15]**

| Host | Strain | OD | Plasmid DNA (ng/µL) | | Proportion (%) ((x1/4)/x1)*100 |
|---|---|---|---|---|---|
| | | | x1 | x1/4 | |
| DH10B | WT | 1.7 | 200.4 | 99.0 | 49 |
| | ΔerfK | 1.8 | 190.4 | 117.2 | 62 |
| | ΔybiS | 1.7 | 219.9 | 116.5 | 53 |
| | ΔycfS | 1.7 | 204.4 | 106.1 | 52 |
| | ΔerfK ΔybiS | 1.7 | 184.5 | 127.5 | 69 |
| | OmpA R256E ΔerfK | 1.7 | 202.1 | 122.0 | 60 |
| | OmpA R256E ΔybiS | 1.7 | 260.7 | 178.0 | 68 |
| | OmpA R256E ΔycfS | 1.6 | 280.6 | 188.8 | 67 |
| | OmpA R256E ΔerfK ΔybiS | 1.8 | 223.8 | 144.3 | 64 |

When comparing plasmid DNA recovered under the x1/4 condition with plasmid DNA recovered under the x1 condition with an extraction efficiency of 100%, WT showed an extraction efficiency of 49%. Meanwhile, the extraction efficiency was higher than WT, with Δ*erfK* at 62%, Δ*ybiS* at 53%, Δ*ycfS* at 52%, ΔerfKΔybiS at 69%, OmpA R256E Δ*erfK* at 60%, OmpA R256E Δ*ybiS* at 68%, OmpA R256E Δ*ycfS* at 67%, and OmpA R256E Δ*erfKΔybiS* at 64%. The extraction efficiency was improved for *ΔybiS* and *ΔycfS* by combining mutations with OmpA R256E and Δ*erfK,* indicating synergistic effects due to the combination of mutations. In addition, the amount of plasmid DNA produced was improved compared to WT in *ΔycfS,* OmpA R256E *ΔybiS,* OmpA R256E *ΔycfS,* and OmpA R256E Δ*erfKΔybiS.*

### [Example 10] Evaluation of LppΔK58 Mutant Strain Using E. coli DH10B Strain as Host

LppΔK58 was prepared by dsDNA recombination of the *E. coli* DH10B strain using the Lambda-Red system. The same operations as in Example 6 were performed for introducing plasmid, culturing, and extracting plasmid. In addition to these mutant strains, the same operations were performed similarly on a control, the DH10B strain (hereafter referred to as "WT"). Table 16 shows the extraction efficiency of the plasmid DNA (mg/L) extracted under each condition compared with the plasmid DNA recovered under the x1 condition with an extraction efficiency of 100%.

**[Table 16]**

| Host | Strain | OD | Plasmid DNA (mg/L) | | Proportion (%) (x2/x1)*100 |
|---|---|---|---|---|---|
| | | | x1 | x2 | |
| DH10B | WT | 4.7 | 11.3±0.4 | 8.3±0.6 | 74 |
| | LppΔK58 | 3.2 | 10.8±0.3 | 9.5±0.5 | 92 |

When comparing plasmid DNA recovered under the x2 condition with plasmid DNA recovered under the x1 condition with an extraction efficiency of 100%, WT showed an extraction efficiency of 74%. Meanwhile, LppΔK58 showed a higher extraction efficiency of 92% than WT. This shows that the above-described mutant strain is a strain from which plasmid DNA can be easily recovered from the bacterial cells.

### [Example 11] Evaluation of L, D-transpeptidase (Ldt) Mutant Strain and Pal+Ldt Double Mutant Strain Using E. coli DH10B Strain as Host

For the *E. coli* DH10B strain, Δ*ybiS* Δ*ycfS,* Δ*erfK* Δ*ybiS* Δ*ycfS, Pal*-s-L14N, *Pal*-s-L14N Δ*erfK,* Pal-s-L14N Δ*ybiS,* Pal-s-L14N Δ*ycfS,* Pal Δ19-43, Pal Δ19-43 Δ*erfK,* Pal Δ19-43 *ΔybiS,* and Pal Δ19-43 Δ*ycfS* were prepared by dsDNA recombination using the Lambda-Red system. The same operations as in Example 6 were performed for introducing plasmid, culturing, and extracting plasmid. In addition to these mutant strains, the same operations were performed similarly on a control, the DH10B strain (hereafter referred to as "WT"). Table 16 shows the extraction efficiency of the plasmid DNA (mg/L) extracted under each condition compared with the plasmid DNA recovered under the x1 condition with an extraction efficiency of 100%.

**[Table 17]**

| Host | Strain | OD | Plasmid DNA (ng/µL) | | Proportion (%) ((x1/4)/x1)*100 |
|---|---|---|---|---|---|
| | | | x1 | x1/4 | |
| DH10B | WT | 2.7 | 118.2 | 49.5 | 42 |
| | ΔybiS ΔycfS | 2.8 | 140.2 | 89.7 | 64 |
| | ΔerfK ΔybiS ΔycfS | 2.4 | 142.8 | 141.3 | 99 |
| | Pal-s-L14N | 2.8 | 141.0 | 112.5 | 80 |
| | Pal-s-L14N ΔerfK | 2.8 | 140.8 | 108.3 | 77 |
| | Pal-s-L14N ΔybiS | 2.7 | 153.9 | 107.6 | 70 |
| | Pal-s-L14N ΔycfS | 2.8 | 143.6 | 121.3 | 84 |
| | Pal Δ19-43 | 2.6 | 154.1 | 109.1 | 71 |
| | Pal Δ19-43 ΔerfK | 2.6 | 135.5 | 96.5 | 71 |
| | Pal Δ19-43 ΔybiS | 2.7 | 129.7 | 101.1 | 78 |
| | Pal Δ19-43 ΔycfS | 2.8 | 124.4 | 101.0 | 81 |

When comparing plasmid DNA recovered under the x2 condition with plasmid DNA recovered under the x1 condition with an extraction efficiency of 100%, WT showed an extraction efficiency of 74%. Meanwhile, the extraction efficiency was higher than WT, with Δ*ybiS* Δ*ycfS* at 64%, Δ*erfK* Δ*ybiS* Δ*ycfS* at 99%, *Pal*-s-L14N at 80%, *Pal*-s-L14N Δ*erfK* at 77%, Pal-s-L14N Δ*ybiS* at 70%, Pal-s-L14N *ΔycfS* at 84%, Pal Δ19-43 at 71%, Pal Δ19-43 Δ*erfK* at 71%, Pal Δ19-43 Δ*ybiS* at 78%, and Pal Δ19-43 Δ*ycfS* at 81%. Synergistic effects on extraction efficiency by the combination of Pal and Ldt were particularly confirmed for Pal-s-L14N Δ*ycfS,* Pal Δ19-43 Δ*ybiS,* and Pal Δ19-43 Δ*ycfS.* In addition, in all mutant strains, the amount of plasmid DNA produced was improved compared to WT.

**[Table 18]**

| SEQ ID NO. | Type | Sequence |
|---|---|---|
| 1 | Structural gene of pal | TGTTCTTCCAACAAGAACGCCAGCAATGACGGCA GCGAAGGCATGCTGGGTGCCGGCACTGGTATGGA TGCGAACGGCGGCAACGGCAACATGTCTTCCGAA GAGCAGGCTCGTCTGCAAATGCAACAGCTGCAGC AGAACAACATCGTTTACTTCGATCTGGACAAGTA CGATATCCGTTCTGACTTCGCTCAAATGCTGGAT GCACATGCAAACTTCCTGCGTAGCAACCCGTCTT ACAAAGTCACCGTAGAAGGTCACGCGGACGAAC GTGGTACTCCGGAATACAACATCTCCCTGGGTGA ACGTCGTGCGAACGCCGTTAAGATGTACCTGCAG GGTAAAGGCGTTTCTGCAGACCAGATCTCCATCG TTTCTTACGGTAAAGAAAAACCTGCAGTACTGGG TCATGACGAAGCGGCATACTCCAAAAACCGTCGT GCGGTACTGGTTTACTAA |
| 2 | Nucleotide sequence of pal signal | ATGCAACTGAACAAAGTGCTGAAAGGGCTGATG ATTGCTCTGCCTGTTATGGCAATTGCGGCA |
| 3 | Structural gene of lpp | TGCTCCAGCAACGCTAAAATCGATCAGCTGTCTT CTGACGTTCAGACTCTGAACGCTAAAGTTGACCA GCTGAGCAACGACGTGAACGCAATGCGTTCCGAC GTTCAGGCTGCTAAAGATGACGCAGCTCGTGCTA ACCAGCGTCTGGACAACATGGCTACTAAATACCG CAAGTAA |
| 4 | Nucleotide sequence of Ipp signal | ATGAAAGCTACTAAACTGGTACTGGGCGCGGTAA TCCTGGGTTCTACTCTGCTGGCAGGT |
| 5 | Structural gene of ompA | GCTCCGAAAGATAACACCTGGTACACTGGTGCTA AACTGGGCTGGTCCCAGTACCATGACACTGGTTT CATCAACAACAATGGCCCGACCCATGAAAACCA ACTGGGCGCTGGTGCTTTTGGTGGTTACCAGGTT AACCCGTATGTTGGCTTTGAAATGGGTTACGACT GGTTAGGTCGTATGCCGTACAAAGGCAGCGTTGA AAACGGTGCATACAAAGCTCAGGGCGTTCAACTG ACCGCTAAACTGGGTTACCCAATCACTGACGACC TGGACATCTACACTCGTCTGGGTGGCATGGTATG GCGTGCAGACACTAAATCCAACGTTTATGGTAAA AACCACGACACCGGCGTTTCTCCGGTCTTCGCTG GCGGTGTTGAGTACGCGATCACTCCTGAAATCGC TACCCGTCTGGAATACCAGTGGACCAACAACATC GGTGACGCACACACCATCGGCACTCGTCCGGACA ACGGCATGCTGAGCCTGGGTGTTTCCTACCGTTTC GGTCAGGGCGAAGCAGCTCCAGTAGTTGCTCCGG |
| | | CTCCAGCTCCGGCACCGGAAGTACAGACCAAGCA CTTCACTCTGAAGTCTGACGTTCTGTTCAACTTCA ACAAAGCAACCCTGAAACCGGAAGGTCAGGCTG CTCTGGATCAGCTGTACAGCCAGCTGAGCAACCT GGATCCGAAAGACGGTTCCGTAGTTGTTCTGGGT TACACCGACCGCATCGGTTCTGACGCTTACAACC AGGGTCTGTCCGAGCGCCGTGCTCAGTCTGTTGT TGATTACCTGATCTCCAAAGGTATCCCGGCAGAC AAGATCTCCGCACGTGGTATGGGCGAATCCAACC CGGTTACTGGCAACACCTGTGACAACGTGAAACA GCGTGCTGCACTGATCGACTGCCTGGCTCCGGAT CGTCGCGTAGAGATCGAAGTTAAAGGTATCAAAG ACGTTGTAACTCAGCCGCAGGCTTAA |
| 6 | Nucleotide sequence of ompA signal | ATGAAAAAGACAGCTATCGCGATTGCAGTGGCAC TGGCTGGTTTCGCTACCGTAGCGCAGGCC |
| 7 | Structural gene of tolA | GTGTCAAAGGCAACCGAACAAAACGACAAGCTC AAGCGGGCGATAATTATTTCAGCAGTGCTGCATG TCATCTTATTTGCGGCGCTGATCTGGAGTTCGTTC GATGAGAATATAGAAGCTTCAGCCGGAGGCGGC GGTGGTTCGTCCATCGACGCTGTCATGGTTGATTC AGGTGCGGTAGTTGAGCAGTACAAACGCATGCA AAGCCAGGAATCAAGCGCGAAGCGTTCTGATGA ACAGCGCAAGATGAAGGAACAGCAGGCTGCTGA AGAACTGCGTGAGAAACAAGCGGCTGAACAGGA ACGCCTGAAGCAACTTGAGAAAGAGCGGTTAGC GGCTCAGGAGCAGAAAAAGCAGGCTGAAGAAGC CGCAAAACAGGCCGAGTTAAAGCAGAAGCAAGC TGAAGAGGCGGCAGCGAAAGCGGCGGCAGATGC TAAAGCGAAGGCCGAAGCAGATGCTAAAGCTGC GGAAGAAGCAGCGAAGAAAGCGGCTGCAGACGC AAAGAAAAAAGCAGAAGCAGAAGCCGCCAAAGC CGCAGCCGAAGCGCAGAAAAAAGCCGAGGCAGC CGCTGCGGCACTGAAGAAGAAAGCGGAAGCGGC AGAAGCAGCTGCAGCTGAAGCAAGAAAGAAAGC GGCAACTGAAGCTGCTGAAAAAGCCAAAGCAGA AGCTGAGAAGAAAGCGGCTGCTGAAAAGGCTGC AGCTGATAAGAAAGCGGCAGCAGAGAAAGCTGC AGCCGACAAAAAAGCAGCAGAAAAAGCGGCTGC TGAAAAGGCAGCAGCTGATAAGAAAGCAGCGGC AGAAAAAGCCGCCGCAGACAAAAAAGCGGCAGC GGCAAAAGCTGCAGCTGAAAAAGCCGCTGCAGC AAAAGCGGCCGCAGAGGCAGATGATATTTTCGGT GAGCTAAGCTCTGGTAAGAATGCACCGAAAACG |
| | | GGGGGAGGGGCGAAAGGGAACAATGCTTCGCCT GCCGGGAGTGGTAATACTAAAAACAATGGCGCA TCAGGGGCCGATATCAATAACTATGCCGGGCAGA TTAAATCTGCTATCGAAAGTAAGTTCTATGACGC ATCGTCCTATGCAGGCAAAACCTGTACGCTGCGC ATAAAACTGGCACCCGATGGTATGTTACTGGATA TCAAACCTGAAGGTGGCGATCCCGCACTTTGTCA GGCTGCGTTGGCAGCAGCTAAACTTGCGAAGATC CCGAAACCACCAAGCCAGGCAGTATATGAAGTGT TCAAAAACGCGCCATTGGACTTCAAACCGTAA |
| 8 | Structural gene of tolB | GAAGTCCGCATTGTGATCGACAGCGGTGTAGATT CCGGTCGTCCTATTGGTGTTGTTCCTTTCCAGTGG GCGGGGCCTGGTGCGGCACCTGAAGATATTGGCG GCATCGTTGCTGCTGACTTGCGTAACAGCGGTAA ATTTAATCCGTTAGATCGCGCTCGTCTGCCACAG CAGCCGGGTAGTGCGCAGGAAGTACAACCAGCT GCATGGTCCGCACTGGGCATTGACGCTGTAGTTG TCGGTCAGGTCACTCCGAATCCGGATGGTTCTTA CAATGTTGCTTATCAACTTGTTGACACTGGCGGC GCACCGGGTACTGTACTTGCTCAGAACTCGTACA AAGTGAACAAGCAGTGGCTGCGTTATGCTGGTCA TACCGCCAGTGATGAAGTGTTTGAAAAACTGACC GGCATTAAAGGTGCGTTCCGTACTCGTATTGCCT ACGTTGTTCAGACCAACGGCGGTCAGTTCCCGTA TGAACTGCGCGTATCTGACTATGACGGTTACAAC CAGTTTGTCGTTCACCGTTCACCGCAGCCGCTGAT GTCTCCGGCGTGGTCACCAGACGGTTCTAAACTG GCTTATGTGACCTTCGAAAGCGGTCGTTCCGCGC TGGTTATTCAGACGCTGGCAAATGGCGCTGTACG TCAGGTGGCTTCATTCCCGCGTCACAACGGTGCA CCTGCATTCTCGCCAGACGGCAGCAAACTGGCAT TCGCCTTGTCGAAAACCGGTAGTCTGAACCTGTA CGTAATGGATTTGGCTTCTGGTCAGATCCGCCAG GTGACTGATGGTCGCAGTAACAATACCGAACCGA CCTGGTTCCCGGATAGCCAGAACCTGGCATTTAC TTCTGACCAGGCCGGTCGTCCGCAGGTTTATAAA GTGAATATCAACGGCGGTGCGCCACAACGTATTA CCTGGGAAGGTTCGCAGAACCAGGATGCGGATGT CAGCAGCGACGGTAAATTTATGGTAATGGTCAGC TCCAATGGTGGGCAGCAGCACATTGCCAAACAAG ATCTGGCAACGGGAGGCGTACAAGTTCTGTCGTC CACGTTCCTGGATGAAACGCCAAGTCTGGCACCT AACGGCACTATGGTAATCTACAGCTCTTCTCAGG |
| | | GGATGGGATCCGTGCTGAATTTGGTTTCTACAGA TGGGCGTTTCAAAGCGCGTCTTCCGGCAACTGAT GGACAGGTCAAATTCCCTGCCTGGTCGCCGTATC TGTGA |
| 9 | Nucleotide sequence of tolB signal | ATGAAGCAGGCATTACGAGTAGCATTTGGTTTTC TCATACTGTGGGCATCAGTTCTGCATGCT |
| 10 | Structural gene of mepS | TGTAGTGCAAATAACACCGCAAAGAATATGCATC CTGAGACACGTGCAGTGGGTAGTGAAACATCATC ACTGCAAGCTTCTCAGGATGAATTTGAAAACCTG GTTCGTAATGTCGACGTAAAATCGCGAATTATGG ATCAGTATGCTGACTGGAAAGGCGTACGTTATCG TCTGGGCGGCAGCACTAAAAAAGGTATCGATTGT TCTGGTTTCGTACAGCGTACATTCCGTGAGCAATT TGGCTTAGAACTTCCGCGTTCGACTTACGAACAG CAGGAAATGGGTAAATCTGTTTCCCGCAGTAATT TGCGTACGGGTGATTTAGTTCTGTTCCGTGCCGGT TCAACGGGACGCCATGTCGGTATTTATATCGGCA ACAATCAGTTTGTCCATGCTTCCACCAGCAGTGG TGTTATTATTTCCAGCATGAATGAACCGTACTGG AAGAAGCGTTACAACGAAGCACGCCGGGTTCTCA GCCGCAGCTAA |
| 11 | Nucleotide sequence of mepS signal | ATGGTCAAATCTCAACCGATTTTGAGATATATCTT GCGCGGGATTCCCGCGATTGCAGTAGCGGTTCTG CTTTCTGCA |
| 12 | Structural gene of nlpI | TGCAGTAATACTTCCTGGCGTAAAAGTGAAGTCC TCGCGGTACCATTGCAACCGACTTTACAGCAGGA AGTGATTCTGGCACGTATGGAACAAATCCTTGCC AGTCGGGCTTTAACCGATGACGAACGCGCACAGC TTTTATATGAGCGCGGAGTGTTGTATGATAGTCTC GGTCTGAGGGCATTAGCGCGTAACGATTTTTCGC AAGCGCTGGCAATCCGACCGGATATGCCTGAAGT ATTCAATTACTTAGGCATATATTTAACGCAGGCA GGCAATTTTGATGCTGCCTATGAAGCGTTTGATTC TGTACTTGAGCTTGATCCAACTTACAACTACGCG CACTTGAATCGCGGGATCGCATTATATTACGGCG GTCGTGACAAGTTAGCGCAAGATGATCTGCTGGC GTTTTATCAAGACGATCCCAATGATCCTTTCCGTA GTCTGTGGCTTTATCTCGCCGAGCAGAAGCTCGA TGAGAAGCAGGCTAAAGAAGTGTTGAAACAGCA CTTCGAAAAATCGGATAAGGAACAGTGGGGATG GAACATTGTCGAGTTCTACCTGGGCAACATTAGC GAACAAACGTTAATGGAAAGGCTCAAGGCGGAC GCAACGGATAACACCTCGCTCGCTGAGCATCTCA |
| | | GTGAAACCAACTTCTATTTAGGTAAGTACTACCT AAGTCTGGGGGATTTGGACAGCGCCACGGCACTG TTCAAACTGGCGGTTGCCAACAACGTTCATAACT TTGTTGAGCACCGATACGCATTGTTGGAATTATC GCTCCTGGGCCAGGACCAAGATGACCTGGCAGA ATCGGACCAGCAATAG |
| 13 | Nucleotide sequence of nlpI signal | ATGAAGCCTTTTTTGCGCTGGTGTTTCGTTGCGAC AGCACTTACGCTTGCAGGA |
| 14 | Structural gene of arcA | ATGCAGACCCCGCACATTCTTATCGTTGAAGACG AGTTGGTAACACGCAACACGTTGAAAAGTATTTT CGAAGCGGAAGGCTATGATGTTTTCGAAGCGACA GATGGCGCGGAAATGCATCAGATCCTCTCTGAAT ATGACATCAACCTGGTGATCATGGATATCAATCT GCCGGGTAAGAACGGTCTTCTGTTAGCGCGTGAA CTGCGCGAGCAGGCGAATGTTGCGTTGATGTTCC TGACTGGCCGTGACAACGAAGTCGATAAAATTCT CGGCCTCGAAATCGGTGCAGATGACTACATCACC AAACCGTTCAACCCGCGTGAACTGACGATTCGTG CACGCAACCTACTGTCCCGTACCATGAATCTGGG TACTGTCAGCGAAGAACGTCGTAGCGTTGAAAGC TACAAGTTCAATGGTTGGGAACTGGACATCAACA GCCGTTCGTTGATCGGCCCTGATGGCGAGCAGTA CAAGCTGCCGCGCAGCGAGTTCCGCGCCATGCTT CACTTCTGTGAAAACCCAGGCAAAATTCAGTCCC GTGCTGAACTGCTGAAGAAAATGACCGGCCGTGA GCTGAAACCGCACGACCGTACTGTAGACGTGACG ATCCGCCGTATTCGTAAACATTTCGAATCTACGC CGGATACGCCGGAAATCATCGCCACCATTCACGG TGAAGGTTATCGCTTCTGCGGTGATCTGGAAGAT TAA |
| 15 | Structural gene of bamD | TGCTCGGGGTCAAAGGAAGAAGTACCTGATAATC CGCCAAATGAAATTTACGCGACTGCACAACAAAA GCTGCAGGACGGTAACTGGAGACAGGCAATAAC GCAACTGGAAGCGTTAGATAATCGCTATCCGTTT GGTCCGTATTCGCAGCAGGTGCAGCTGGATCTCA TCTACGCCTACTATAAAAACGCCGATTTGCCGTT AGCACAGGCTGCCATCGATCGTTTTATTCGCCTTA ACCCGACCCATCCGAATATCGATTATGTCATGTA CATGCGTGGCCTGACCAATATGGCGCTGGATGAC AGTGCGCTGCAAGGGTTCTTTGGCGTCGATCGTA GCGATCGCGATCCTCAACATGCACGAGCTGCGTT TAGTGACTTTTCCAAACTGGTGCGCGGCTATCCG AACAGTCAGTACACCACCGATGCCACCAAACGTC |
| | | TGGTATTCCTGAAAGATCGTCTGGCGAAATATGA ATACTCCGTGGCCGAGTACTATACAGAACGTGGC GCATGGGTTGCCGTCGTTAACCGCGTAGAAGGCA TGTTGCGCGACTACCCGGATACCCAGGCTACGCG TGATGCGCTGCCGCTGATGGAAAATGCATACCGT CAGATGCAGATGAATGCGCAAGCTGAAAAAGTA GCGAAAATCATCGCCGCAAACAGCAGCAATACA TAA |
| 16 | Nucleotide sequence of bamD signal | ATGACGCGCATGAAATATCTGGTGGCAGCCGCCA CACTAAGCCTGTTTTTGGCGGGT |
| 17 | Structural gene of cyoA | TGTAATTCTGCGCTGTTAGATCCCAAAGGACAGA TTGGTCTGGAGCAACGTTCACTGATACTGACGGC ATTTGGCCTGATGTTGATTGTCGTTATTCCCGCAA TCTTGATGGCTGTTGGTTTCGCCTGGAAGTACCGT GCGAGCAATAAAGATGCTAAGTACAGCCCGAAC TGGTCACACTCCAATAAAGTGGAAGCTGTGGTCT GGACGGTACCTATCTTAATCATCATCTTCCTTGCA GTACTGACCTGGAAAACCACTCACGCTCTTGAGC CTAGCAAGCCGCTGGCACACGACGAGAAGCCCA TTACCATCGAAGTGGTTTCCATGGACTGGAAATG GTTCTTCATCTACCCGGAACAGGGCATTGCTACC GTGAATGAAATCGCTTTCCCGGCGAACACTCCGG TGTACTTCAAAGTGACCTCCAACTCCGTGATGAA CTCCTTCTTCATTCCGCGTCTGGGTAGCCAGATTT ATGCCATGGCCGGTATGCAGACTCGCCTGCATCT GATCGCCAACGAACCCGGCACTTATGACGGTATC TCCGCCAGCTACAGCGGCCCGGGCTTCTCAGGCA TGAAGTTCAAAGCTATTGCAACACCGGATCGCGC CGCATTCGACCAGTGGGTCGCAAAAGCGAAGCA GTCGCCGAACACCATGTCTGACATGGCTGCGTTC GAAAAACTGGCCGCGCCTAGCGAATACAACCAG GTGGAATATTTCTCCAACGTGAAACCAGACTTGT TTGCCGATGTAATTAACAAGTTTATGGCTCACGG TAAGAGCATGGACATGACCCAGCCAGAAGGTGA GCACAGCGCACACGAAGGTATGGAAGGCATGGA CATGAGCCACGCGGAATCCGCCCATTAA |
| 18 | Nucleotide sequence of cyoA signal | ATGAGACTCAGGAAATACAATAAAAGTTTGGGAT GGTTGTCATTATTTGCAGGCACTGTATTGCTCAGT GGC |
| 19 | Structural gene of dppA | AAAACTCTGGTTTATTGCTCAGAAGGATCTCCGG AAGGGTTTAACCCGCAGCTGTTTACCTCCGGCAC CACCTATGACGCCTCTTCCGTCCCGCTTTATAACC GTCTGGTTGAATTTAAAATCGGCACCACCGAAGT |
| | | GATCCCGGGCCTCGCTGAAAAGTGGGAAGTCAGC GAAGACGGTAAAACCTATACCTTCCATCTGCGTA AAGGTGTGAAGTGGCACGACAATAAAGAATTCA AACCGACGCGTGAACTGAACGCCGATGATGTGGT GTTCTCGTTCGATCGTCAGAAAAACGCGCAAAAC CCGTACCATAAAGTTTCTGGCGGCAGCTACGAAT ACTTCGAAGGCATGGGCTTGCCAGAGCTGATCAG TGAAGTGAAAAAGGTGGACGACAACACCGTTCA GTTTGTGCTGACTCGCCCGGAAGCGCCGTTCCTC GCTGACCTGGCAATGGACTTCGCCTCTATTCTGTC AAAAGAATATGCTGATGCGATGATGAAAGCCGG TACACCGGAAAAACTGGACCTCAACCCAATCGGA ACCGGTCCGTTCCAGTTACAGCAGTATCAAAAAG ATTCCCGTATCCGCTACAAAGCGTTTGATGGCTA CTGGGGCACCAAACCGCAGATCGATACGCTGGTT TTCTCTATTACCCCTGACGCTTCCGTGCGTTACGC GAAATTGCAGAAGAATGAATGCCAGGTGATGCC GTACCCGAACCCGGCAGATATCGCTCGCATGAAG CAGGATAAATCCATCAATCTGATGGAAATGCCGG GGCTGAACGTCGGTTATCTCTCGTATAACGTGCA GAAAAAACCACTCGATGACGTGAAAGTTCGCCA GGCTCTGACCTACGCGGTGAACAAAGACGCGATC ATCAAAGCGGTTTATCAGGGCGCGGGCGTATCAG CGAAAAACCTGATCCCGCCAACCATGTGGGGCTA TAACGACGACGTTCAGGACTACACCTACGATCCT GAAAAAGCGAAAGCCTTGCTGAAAGAAGCGGGT CTGGAAAAAGGTTTCTCCATCGACCTGTGGGCGA TGCCGGTACAACGTCCGTATAACCCGAACGCTCG CCGCATGGCGGAGATGATTCAGGCAGACTGGGC GAAAGTCGGCGTGCAGGCCAAAATTGTCACCTAC GAATGGGGTGAGTACCTCAAGCGTGCGAAAGAT GGCGAGCACCAGACGGTAATGATGGGCTGGACT GGCGATAACGGGGATCCGGATAACTTCTTCGCCA CCCTGTTCAGCTGCGCCGCCTCTGAACAAGGCTC CAACTACTCAAAATGGTGCTACAAACCGTTTGAA GATCTGATTCAACCGGCGCGTGCTACCGACGACC ACAATAAACGCGTTGAACTGTACAAACAAGCGC AGGTGGTGATGCACGATCAGGCTCCGGCACTGAT CATCGCTCACTCCACCGTGTTTGAACCGGTACGT AAAGAAGTTAAAGGCTATGTGGTTGATCCATTAG GCAAACATCACTTCGAAAACGTCTCTATCGAATA A |
| 20 | Nucleotide sequence of dppA signal | ATGCGTATTTCCTTGAAAAAGTCAGGGATGCTGA AGCTTGGTCTCAGCCTGGTGGCTATGACCGTCGC AGCAAGTGTTCAGGCT |
| 21 | Structural gene of ecnB | TGCAACACCACGCGTGGCGTTGGTGAAGACATTT CTGATGGCGGTAACGCGATTTCTGGCGCAGCAAC GAAAGCGCAGCAATAA |
| 22 | Nucleotide sequence of ecnB signal | ATGGTGAAGAAGACAATTGCAGCGATCTTTTCTG TTCTGGTGCTTTCAACAGTATTAACTGCC |
| 23 | Structural gene of mrcA | GTGAAGTTCGTAAAGTATTTTTTGATCCTTGCAGT CTGTTGCATTCTGCTGGGAGCAGGCTCGATTTAT GGCCTATACCGCTACATCGAGCCACAACTGCCGG ATGTGGCGACATTAAAAGATGTTCGCCTGCAAAT TCCGATGCAGATTTACAGCGCCGATGGCGAGCTG ATTGCTCAATACGGTGAGAAACGTCGTATTCCGG TTACGTTGGATCAAATCCCACCGGAGATGGTGAA AGCCTTTATCGCGACAGAAGACAGCCGCTTCTAC GAGCATCACGGCGTTGACCCGGTGGGGATCTTCC GTGCAGCAAGCGTGGCGCTGTTCTCCGGTCACGC GTCACAAGGGGCAAGTACCATTACCCAGCAGCTG GCGAGAAACTTCTTCCTCAGTCCAGAACGCACGC TGATGCGTAAGATTAAGGAAGTCTTCCTCGCGAT TCGCATTGAACAGCTGCTGACGAAAGACGAGATC CTCGAGCTTTATCTGAACAAGATTTACCTTGGTTA CCGCGCCTATGGTGTCGGTGCTGCGGCACAAGTC TATTTCGGAAAAACGGTCGACCAACTGACGCTGA ACGAAATGGCGGTGATAGCCGGGCTGCCGAAAG CGCCTTCCACCTTCAACCCGCTCTACTCGATGGAT CGTGCCGTCGCGCGGCGTAACGTCGTGCTGTCGC GGATGCTGGATGAAGGGTATATCACCCAACAACA GTTCGATCAGACACGCACTGAGGCGATTAACGCT AACTATCACGCGCCGGAGATTGCTTTCTCTGCGC CGTACCTGAGCGAAATGGTGCGCCAGGAGATGTA TAACCGTTATGGCGAAAGTGCCTATGAAGACGGT TATCGCATTTACACCACCATCACCCGCAAAGTGC AGCAGGCCGCGCAGCAGGCGGTACGTAATAACG TGCTGGACTACGACATGCGCCACGGCTATCGCGG CCCGGCAAATGTGCTGTGGAAAGTGGGCGAGTCG GCGTGGGATAACAACAAGATTACCGATACGCTGA AGGCGCTGCCAACCTATGGTCCGCTGCTGCCTGC CGCAGTCACCAGCGCCAATCCTCAGCAAGCGACG GCGATGCTGGCGGACGGGTCGACCGTCGCATTGA GTATGGAAGGCGTTCGCTGGGCGCGTCCTTACCG TTCGGATACTCAGCAAGGACCGACGCCGCGTAAA |
| | | GTGACCGATGTTCTGCAAACGGGTCAGCAAATCT GGGTTCGTCAGGTTGGCGATGCATGGTGGCTGGC ACAAGTGCCGGAAGTGAACTCGGCGCTGGTGTCG ATCAATCCGCAAAACGGTGCCGTTATGGCGCTGG TCGGTGGCTTTGATTTCAATCAGAGCAAGTTTAA CCGCGCCACCCAGGCACTGCGTCAGGTGGGTTCC AACATCAAACCGTTCCTCTACACCGCGGCGATGG ATAAAGGTCTGACGCTGGCAAGTATGTTGAACGA TGTGCCAATTTCTCGCTGGGATGCAAGTGCCGGT TCTGACTGGCAGCCGAAGAACTCACCACCGCAGT ATGCTGGTCCAATTCGCTTACGTCAGGGGCTGGG TCAGTCGAAAAACGTGGTGATGGTACGCGCAATG CGGGCGATGGGCGTCGACTACGCTGCAGAATATC TGCAACGCTTCGGCTTCCCGGCACAAAACATTGT CCACACCGAATCGCTGGCGCTGGGTTCAGCGTCC TTCACCCCAATGCAGGTGGCGCGCGGCTACGCGG TCATGGCGAACGGCGGCTTCCTGGTGGACCCGTG GTTTATCAGCAAAATTGAAAACGATCAGGGCGGC GTGATTTTCGAAGCGAAACCGAAAGTAGCCTGCC CGGAATGCGATATTCCGGTGATTTACGGTGATAC GCAGAAATCGAACGTGCTGGAAAATAACGATGTT GAAGATGTCGCTATCTCCCGCGAGCAGCAGAATG TTTCTGTACCAATGCCGCAGCTGGAGCAGGCAAA TCAGGCGTTAGTGGCGAAGACTGGCGCGCAGGA GTACGCACCGCACGTCATCAACACTCCGCTGGCA TTCCTGATTAAGAGTGCTTTGAACACCAATATCTT TGGTGAGCCAGGCTGGCAGGGTACTGGCTGGCGT GCAGGTCGTGATTTGCAGCGTCGCGATATCGGCG GGAAAACCGGGACCACTAACAGTTCGAAAGATG CGTGGTTCTCGGGTTACGGTCCGGGCGTTGTGAC CTCGGTCTGGATTGGCTTTGATGATCACCGTCGTA ATCTCGGTCATACAACGGCTTCCGGAGCGATTAA AGATCAGATCTCAGGTTACGAAGGCGGTGCCAAG AGTGCCCAGCCTGCATGGGACGCTTATATGAAAG CCGTTCTTGAAGGTGTGCCGGAGCAGCCGCTGAC GCCGCCACCGGGTATTGTGACGGTGAATATCGAT CGCAGCACCGGGCAGTTAGCTAATGGTGGCAACA GCCGCGAAGAGTATTTCATCGAAGGTACGCAGCC GACACAACAGGCAGTGCACGAGGTGGGAACGAC CATTATCGATAATGGCGAGGCACAGGAATTGTTC TGA |
| 24 | Structural gene of mrcB | ATGGCCGGGAATGACCGCGAGCCAATTGGACGC AAAGGGAAACCGACGCGTCCGGTCAAACAAAAG |
| | | GTAAGCCGTCGTCGTTACGAAGATGACGATGATT ACGACGATTATGATGACTATGAGGATGAAGAACC GATGCCGCGCAAAGGTAAGGGCAAAGGCAAAGG GCGTAAGCCTCGTGGCAAACGCGGCTGGCTATGG CTACTGCTAAAACTGGCTATCGTTTTTGCCGTGCT GATCGCCATTTACGGCGTTTATCTCGATCAAAAA ATTCGTAGCCGTATTGATGGCAAGGTCTGGCAAC TGCCTGCGGCAGTTTATGGCCGAATGGTCAATCT TGAGCCAGACATGACCATCAGCAAGAACGAGAT GGTGAAGCTGCTGGAGGCGACCCAGTATCGTCAG GTGTCGAAAATGACCCGTCCTGGCGAATTTACCG TGCAGGCCAACAGCATTGAGATGATTCGCCGTCC GTTTGATTTCCCGGACAGTAAAGAAGGACAGGTG CGCGCGCGTCTGACCTTTGATGGCGATCATCTGG CGACGATCGTCAATATGGAGAACAACCGTCAGTT CGGTTTCTTCCGTCTTGATCCGCGTCTGATCACCA TGATCTCTTCGCCAAACGGTGAGCAGCGTCTGTT TGTGCCGCGCAGTGGTTTCCCGGATTTGCTGGTG GATACTTTGCTGGCGACAGAAGACCGTCATTTTT ACGAGCATGATGGAATCAGTCTCTACTCAATCGG ACGTGCGGTGCTGGCAAACCTGACCGCCGGACGC ACGGTACAGGGTGCGAGTACGCTGACGCAACAG CTGGTGAAAAACCTGTTCCTCTCCAGCGAGCGTT CTTACTGGCGTAAAGCGAACGAAGCTTACATGGC GCTGATCATGGACGCGCGTTACAGCAAAGACCGT ATTCTTGAGCTGTATATGAACGAGGTGTATCTCG GTCAGAGCGGCGACAACGAAATCCGCGGCTTCCC GCTGGCAAGCTTGTATTACTTTGGTCGCCCGGTA GAAGAGCTAAGCCTCGACCAGCAGGCGCTGTTAG TCGGTATGGTGAAAGGGGCGTCCATCTACAACCC GTGGCGTAACCCAAAACTGGCGCTGGAGCGACGT AATCTGGTGCTGCGTCTGCTGCAACAGCAACAGA TTATTGATCAAGAACTCTATGACATGTTGAGTGC CCGTCCGCTGGGGGTTCAGCCGCGCGGTGGGGTG ATCTCTCCTCAGCCAGCCTTTATGCAACTGGTGCG TCAGGAGCTGCAGGCAAAACTGGGCGATAAGGT AAAAGATCTCTCCGGCGTGAAGATCTTCACTACC TTTGACTCGGTGGCCCAGGACGCGGCAGAAAAA GCCGCCGTGGAAGGCATTCCGGCACTGAAGAAA CAGCGTAAGTTGAGCGATCTTGAAACTGCGATTG TGGTCGTCGACCGCTTTAGTGGTGAAGTTCGTGC GATGGTCGGAGGTTCTGAGCCGCAGTTTGCGGGC TACAACCGTGCGATGCAGGCGCGTCGTTCGATTG |
| | | GTTCCCTTGCAAAACCAGCGACTTATCTGACGGC CTTAAGCCAGCCGAAAATCTATCGTCTGAATACG TGGATTGCGGATGCGCCAATTGCGCTGCGTCAGC CGAATGGCCAGGTCTGGTCACCGCAGAATGATGA CCGTCGTTATAGCGAAAGCGGCAGAGTGATGCTG GTGGATGCGTTGACCCGTTCGATGAACGTGCCGA CGGTAAATCTGGGGATGGCGCTGGGGCTGCCTGC GGTTACGGAGACCTGGATTAAACTGGGCGTACCG AAAGATCAGTTGCATCCGGTTCCGGCAATGCTGC TGGGGGCGTTGAACTTAACGCCAATCGAAGTGGC GCAGGCATTCCAGACCATCGCCAGCGGTGGTAAC CGTGCACCGCTTTCTGCGCTGCGTTCGGTAATCGC GGAAGATGGCAAAGTGCTGTATCAGAGCTTCCCG CAGGCGGAACGCGCTGTTCCGGCGCAGGCGGCGT ATCTGACACTATGGACCATGCAGCAGGTGGTACA ACGCGGTACGGGTCGTCAGCTTGGGGCGAAATAC CCGAACCTGCATCTGGCAGGGAAAACAGGGACT ACCAACAATAACGTAGATACCTGGTTTGCGGGCA TTGACGGCAGCACGGTGACCATCACCTGGGTCGG CCGTGATAACAACCAGCCGACCAAACTGTATGGT GCCAGCGGGGCAATGTCGATTTATCAGCGTTATC TGGCTAACCAGACGCCAACGCCGCTGAATCTTGT TCCGCCAGAAGATATTGCAGATATGGGCGTGGAC TACGACGGCAACTTTGTTTGCAGCGGTGGCATGC GTATCTTGCCGGTCTGGACCAGCGATCCGCAATC GCTGTGCCAGCAGAGCGAGATGCAGCAGCAGCC GTCAGGCAATCCGTTTGATCAGTCTTCTCAGCCG CAGCAACAGCCGCAACAGCAACCTGCTCAGCAA GAGCAGAAAGACAGCGACGGTGTAGCCGGTTGG ATCAAGGATATGTTTGGTAGTAATTAA |
| 25 | Structural gene of oppA | GCTGATGTACCCGCAGGCGTCACACTGGCGGAAA AACAAACACTGGTACGTAACAATGGTTCAGAAGT TCAGTCATTAGATCCGCACAAAATTGAAGGTGTT CCGGAGTCTAATATCAGCCGAGACCTGTTTGAAG GCTTACTGGTCAGCGATCTTGACGGTCATCCAGC ACCTGGCGTCGCTGAATCCTGGGATAATAAAGAC GCGAAAGTCTGGACCTTCCATTTGCGTAAAGATG CGAAATGGTCTGATGGCACGCCAGTCACAGCACA AGACTTTGTGTATAGCTGGCAACGTTCTGTTGATC CGAACACTGCTTCTCCGTATGCCAGTTATCTGCA ATATGGGCATATCGCCGGTATTGATGAAATTCTT GAAGGGAAAAAACCGATTACCGATCTCGGCGTG AAAGCTATTGATGATCACACATTAGAAGTCACCT |
| | | TAAGTGAACCCGTTCCGTACTTCTATAAATTACTT GTTCACCCATCAACTTCACCGGTGCCAAAAGCCG CTATCGAGAAATTCGGCGAAAAATGGACCCAGCC TGGTAATATCGTCACCAACGGTGCCTATACCTTA AAAGATTGGGTCGTAAACGAACGAATCGTTCTTG AACGCAGCCCGACCTACTGGAACAACGCGAAAA CCGTTATTAACCAGGTAACCTATTTGCCTATTGCT TCTGAAGTTACCGATGTCAACCGCTACCGTAGTG GTGAAATCGACATGACGAATAACAGCATGCCGAT CGAATTGTTCCAGAAGCTGAAAAAAGAGATCCCG GACGAAGTTCACGTTGATCCATACCTGTGCACTT ACTATTACGAAATTAACAACCAGAAACCGCCATT CAACGATGTGCGTGTGCGTACCGCACTGAAACTA GGTATGGACCGCGATATCATTGTTAATAAAGTGA AAGCGCAGGGCAACATGCCCGCCTATGGTTACAC TCCACCGTATACTGATGGCGCAAAATTGACTCAG CCAGAATGGTTTGGCTGGAGCCAGGAAAAACGT AACGAAGAAGCGAAAAAACTGCTGGCTGAAGCG GGTTATACCGCAGACAAACCGTTGACCATCAACC TGTTGTATAACACCTCCGATCTGCATAAAAAGCT GGCGATTGCTGCCTCTTCATTGTGGAAGAAAAAC ATTGGTGTAAACGTCAAACTGGTTAACCAGGAGT GGAAAACGTTCCTCGACACCCGTCACCAGGGTAC TTTTGATGTGGCCCGTGCAGGCTGGTGTGCTGAC TACAACGAACCAACTTCCTTCCTGAACACCATGC TTTCGAACAGCTCGATGAATACCGCGCATTATAA GAGCCCGGCCTTTGACAGCATTATGGCGGAAACG CTGAAAGTGACTGACGAGGCGCAGCGCACAGCT CTGTACACTAAAGCAGAACAACAGCTGGATAAG GATTCGGCCATTGTTCCTGTTTATTACTACGTGAA TGCGCGTCTGGTGAAACCGTGGGTTGGTGGCTAT ACCGGCAAAGATCCGCTGGATAATACCTATACCC GGAATATGTACATTGTGAAGCACTAA |
| 26 | Nucleotide sequence of oppA signal | ATGACCAACATCACCAAGAGAAGTTTAGTAGCAG CTGGCGTTCTGGCTGCGCTAATGGCAGGGAATGT CGCGCTGGCA |
| 27 | Structural gene of slyB | TGTGTTAATAACGACACCCTGTCAGGGGATGTTT ATACCGCTTCTGAAGCGAAACAAGTACAGAATGT CAGCTATGGCACCATCGTTAACGTACGTCCGGTA CAGATTCAGGGCGGTGATGATTCCAACGTTATCG GTGCAATTGGCGGTGCTGTTCTTGGTGGTTTCCTG GGGAATACTGTTGGTGGCGGAACCGGGCGTTCTC TGGCTACTGCAGCAGGCGCTGTTGCAGGTGGCGT |
| | | AGCTGGTCAGGGCGTACAGAGTGCAATGAACAA AACGCAGGGTGTCGAGCTGGAAATTCGTAAAGA CGATGGTAATACCATCATGGTGGTACAGAAACAA GGCAACACTCGTTTCTCTCCGGGCCAACGTGTCG TACTGGCCAGCAATGGCAGTCAGGTGACCGTTTC TCCGCGCTAA |
| 28 | Nucleotide sequence of slyB signal | ATGATTAAACGCGTATTGGTTGTTTCAATGGTAG GTCTGTCTCTTGTCGGT |
| 29 | Structural gene of tolQ | GTGACTGACATGAATATCCTTGATTTGTTCCTGAA GGCTAGCCTTCTGGTTAAACTTATCATGTTGATTT TGATTGGTTTTTCAATCGCATCTTGGGCCATTATT ATCCAGCGGACCCGTATTCTTAACGCAGCGGCGC GCGAAGCCGAAGCGTTTGAAGATAAATTCTGGTC TGGAATCGAACTCTCTCGCCTCTATCAAGAGAGC CAGGGGAAACGGGATAATCTGACTGGTTCGGAA CAAATCTTTTACAGCGGGTTCAAAGAGTTTGTGC GCCTGCATCGTGCCAATAGCCATGCGCCGGAAGC CGTAGTGGAAGGGGCGTCGCGTGCTATGCGTATC TCCATGAACCGTGAACTTGAAAATCTGGAAACGC ACATTCCGTTCCTCGGTACGGTTGGCTCCATCAGC CCGTATATTGGTCTGTTTGGTACGGTCTGGGGGA TCATGCACGCCTTTATCGCCCTCGGGGCGGTAAA ACAAGCAACACTGCAAATGGTTGCGCCCGGTATC GCAGAAGCGTTGATTGCGACTGCAATTGGTCTGT TTGCCGCTATCCCGGCAGTTATGGCCTACAACCG CCTCAACCAGCGCGTAAACAAACTGGAACTGAAT TACGACAACTTTATGGAAGAGTTTACCGCGATTC TGCACCGCCAGGCGTTTACCGTTAGCGAGAGCAA CAAGGGGTAA |
| 30 | Structural gene of tolR | ATGGCCAGAGCGCGTGGACGAGGTCGTCGCGATC TCAAGTCCGAAATCAACATTGTACCGTTGCTGGA CGTACTGCTGGTGCTGTTGCTGATCTTTATGGCGA CAGCGCCCATCATCACCCAGAGCGTGGAGGTCGA TCTGCCAGACGCTACTGAATCACAGGCGGTGAGC AGTAACGATAATCCGCCAGTGATTGTTGAAGTGT CTGGTATTGGTCAGTACACCGTGGTGGTTGAGAA AGATCGCCTGGAGCGTTTACCACCAGAGCAGGTG GTGGCGGAAGTGTCCAGCCGTTTCAAGGCCAACC CGAAAACGGTCTTTCTGATCGGTGGCGCAAAAGA TGTGCCTTACGATGAAATAATTAAAGCACTGAAC TTGTTACATAGTGCGGGTGTGAAATCGGTTGGTT TAATGACGCAGCCTATCTAA |
| 31 | Pal | CSSNKNASNDGSEGMLGAGTGMDANGGNGNMSS EEQARLQMQQLQQNNIVYFDLDKYDIRSDFAQML DAHANFLRSNPSYKVTVEGHADERGTPEYNISLGE RRANAVKMYLQGKGVSADQISIVSYGKEKPAVLG HDEAAYSKNRRAVLVY |
| 32 | Pal signal peptide | MQLNKVLKGLMIALPVMAIAA |
| 33 | Pal-deficient sequence (Amino acid sequence) | CSAGTGMDANGGNGNMSSEEQARLQMQQLQQNNI VYFDLDKYDIRSDFAQMLDAHANFLRSNPSYKVTV EGHADERGTPEYNISLGERRANAVKMYLQGKGVS ADQISIVSYGKEKPAVLGHDEAAYSKNRRAVLVY |
| 34 | Pal-deficient sequence (Nucleotide sequence) | TGTTCTGCCGGCACTGGTATGGATGCGAACGGCG GCAACGGCAACATGTCTTCCGAAGAGCAGGCTCG TCTGCAAATGCAACAGCTGCAGCAGAACAACATC GTTTACTTCGATCTGGACAAGTACGATATCCGTTC TGACTTCGCTCAAATGCTGGATGCACATGCAAAC TTCCTGCGTAGCAACCCGTCTTACAAAGTCACCG TAGAAGGTCACGCGGACGAACGTGGTACTCCGG AATACAACATCTCCCTGGGTGAACGTCGTGCGAA CGCCGTTAAGATGTACCTGCAGGGTAAAGGCGTT TCTGCAGACCAGATCTCCATCGTTTCTTACGGTAA AGAAAAACCTGCAGTACTGGGTCATGACGAAGC GGCATACTCCAAAAACCGTCGTGCGGTACTGGTT TACTAA |
| 35 | Pal-deficient sequence (Amino acid sequence) | CSSNKNASNDGSEGMLGALQQNNIVYFDLDKYDIR SDFAQMLDAHANFLRSNPSYKVTVEGHADERGTPE YNISLGERRANAVKMYLQGKGVSADQISIVSYGKE KPAVLGHDEAAYSKNRRAVLVY |
| 36 | Pal-deficient sequence (Nucleotide sequence) | TGTTCTTCCAACAAGAACGCCAGCAATGACGGCA GCGAAGGCATGCTGGGTGCCCTGCAGCAGAACA ACATCGTTTACTTCGATCTGGACAAGTACGATAT CCGTTCTGACTTCGCTCAAATGCTGGATGCACAT GCAAACTTCCTGCGTAGCAACCCGTCTTACAAAG TCACCGTAGAAGGTCACGCGGACGAACGTGGTAC TCCGGAATACAACATCTCCCTGGGTGAACGTCGT GCGAACGCCGTTAAGATGTACCTGCAGGGTAAAG GCGTTTCTGCAGACCAGATCTCCATCGTTTCTTAC GGTAAAGAAAAACCTGCAGTACTGGGTCATGAC GAAGCGGCATACTCCAAAAACCGTCGTGCGGTAC TGGTTTACTAA |
| 37 | Pal-deficient sequence (Amino acid sequence) | CSSNKNASNDGSEGMLGAGTGMDANGGNGNMSS EEQARLQMQQFAQMLDAHANFLRSNPSYKVTVEG HADERGTPEYNISLGERRANAVKMYLQGKGVSAD QISIVSYGKEKPAVLGHDEAAYSKNRRAVLVY |
| 38 | Pal-deficient sequence (Nucleotide sequence) | TGTTCTTCCAACAAGAACGCCAGCAATGACGGCA GCGAAGGCATGCTGGGTGCCGGCACTGGTATGGA TGCGAACGGCGGCAACGGCAACATGTCTTCCGAA GAGCAGGCTCGTCTGCAAATGCAACAGTTCGCTC AAATGCTGGATGCACATGCAAACTTCCTGCGTAG CAACCCGTCTTACAAAGTCACCGTAGAAGGTCAC GCGGACGAACGTGGTACTCCGGAATACAACATCT CCCTGGGTGAACGTCGTGCGAACGCCGTTAAGAT GTACCTGCAGGGTAAAGGCGTTTCTGCAGACCAG ATCTCCATCGTTTCTTACGGTAAAGAAAAACCTG CAGTACTGGGTCATGACGAAGCGGCATACTCCAA AAACCGTCGTGCGGTACTGGTTTACTAA |
| 39 | Pal-deficient sequence (Amino acid sequence) | CSSNKNASNDGSEGMLGAGTGMDANGGNGNMSS EEQARLQMQQLQQNNIVYFDLDKYDIRSPEYNISLG ERRANAVKMYLQGKGVSADQISIVSYGKEKPAVLG HDEAAYSKNRRAVLVY |
| 40 | Pal-deficient sequence (Nucleotide sequence) | TGTTCTTCCAACAAGAACGCCAGCAATGACGGCA GCGAAGGCATGCTGGGTGCCGGCACTGGTATGGA TGCGAACGGCGGCAACGGCAACATGTCTTCCGAA GAGCAGGCTCGTCTGCAAATGCAACAGCTGCAGC AGAACAACATCGTTTACTTCGATCTGGACAAGTA CGATATCCGTTCTCCGGAATACAACATCTCCCTG GGTGAACGTCGTGCGAACGCCGTTAAGATGTACC TGCAGGGTAAAGGCGTTTCTGCAGACCAGATCTC CATCGTTTCTTACGGTAAAGAAAAACCTGCAGTA CTGGGTCATGACGAAGCGGCATACTCCAAAAACC GTCGTGCGGTACTGGTTTACTAA |
| 41 | Pal-deficient sequence (Amino acid sequence) | CSSNKNASNDGSEGMLGAGTGMDANGGNGNMSS EEQARLQMQQLQQNNIVYFDLDKYDIRSDFAQML DAHANFLRSNPSYKVTVEGHADERGTISIVSYGKEK PAVLGHDEAAYSKNRRAVLVY |
| 42 | Pal-deficient sequence (Nucleotide sequence) | TGTTCTTCCAACAAGAACGCCAGCAATGACGGCA GCGAAGGCATGCTGGGTGCCGGCACTGGTATGGA TGCGAACGGCGGCAACGGCAACATGTCTTCCGAA GAGCAGGCTCGTCTGCAAATGCAACAGCTGCAGC AGAACAACATCGTTTACTTCGATCTGGACAAGTA CGATATCCGTTCTGACTTCGCTCAAATGCTGGAT GCACATGCAAACTTCCTGCGTAGCAACCCGTCTT ACAAAGTCACCGTAGAAGGTCACGCGGACGAAC GTGGTACTATCTCCATCGTTTCTTACGGTAAAGA AAAACCTGCAGTACTGGGTCATGACGAAGCGGC ATACTCCAAAAACCGTCGTGCGGTACTGGTTTAC TAA |
| 43 | Pal-deficient sequence (Amino acid sequence) | CSSNKNASNDGSEGMLGAGTGMDANGGNGNMSS EEQARLQMQQLQQNNIVYFDLDKYDIRSDFAQML DAHANFLRSNPSYKVTVEGHADERGTPEYNISLGE RRANAVKMYLQGKGVSADQI |
| 44 | Pal-deficient sequence (Nucleotide sequence) | TGTTCTTCCAACAAGAACGCCAGCAATGACGGCA GCGAAGGCATGCTGGGTGCCGGCACTGGTATGGA TGCGAACGGCGGCAACGGCAACATGTCTTCCGAA GAGCAGGCTCGTCTGCAAATGCAACAGCTGCAGC AGAACAACATCGTTTACTTCGATCTGGACAAGTA CGATATCCGTTCTGACTTCGCTCAAATGCTGGAT GCACATGCAAACTTCCTGCGTAGCAACCCGTCTT ACAAAGTCACCGTAGAAGGTCACGCGGACGAAC GTGGTACTCCGGAATACAACATCTCCCTGGGTGA ACGTCGTGCGAACGCCGTTAAGATGTACCTGCAG GGTAAAGGCGTTTCTGCAGACCAGATCTAA |
| 45 | Pal-deficient sequence (Amino acid sequence) | CSSNKNASNDGSEGMLGAGTGMDANGGNGNMSS EEQARLQMQQLQQNNIVYFDLDKYDIRSDFAQML DAHANFLRSNPSYKVTVEGHADERGTPEYNISLGE RRANAVKMYLQGKGVSADQISIVEKPAVLGHDEA AYSKNRRAVLVY |
| 46 | Pal-deficient sequence (Nucleotide sequence) | TGTTCTTCCAACAAGAACGCCAGCAATGACGGCA GCGAAGGCATGCTGGGTGCCGGCACTGGTATGGA TGCGAACGGCGGCAACGGCAACATGTCTTCCGAA GAGCAGGCTCGTCTGCAAATGCAACAGCTGCAGC AGAACAACATCGTTTACTTCGATCTGGACAAGTA CGATATCCGTTCTGACTTCGCTCAAATGCTGGAT GCACATGCAAACTTCCTGCGTAGCAACCCGTCTT ACAAAGTCACCGTAGAAGGTCACGCGGACGAAC GTGGTACTCCGGAATACAACATCTCCCTGGGTGA ACGTCGTGCGAACGCCGTTAAGATGTACCTGCAG GGTAAAGGCGTTTCTGCAGACCAGATCTCCATCG TTGAAAAACCTGCAGTACTGGGTCATGACGAAGC GGCATACTCCAAAAACCGTCGTGCGGTACTGGTT TACTAA |
| 47 | Pal-deficient sequence (Amino acid sequence) | CSSNKNASNDGSEGMLGAGTGMDANGGNGNMSS EEQARLQMQQLQQNNIVYFDLDKYDIRSDFAQML DAHANFLRSNPSYKVTVEGHADERGTPEYNISLGE RRANAVKMYLQGKGVSADQISIVSYGKEKPAVLG HDEAAYSAVLVY |
| 48 | Pal-deficient sequence (Nucleotide sequence) | TGTTCTTCCAACAAGAACGCCAGCAATGACGGCA GCGAAGGCATGCTGGGTGCCGGCACTGGTATGGA TGCGAACGGCGGCAACGGCAACATGTCTTCCGAA GAGCAGGCTCGTCTGCAAATGCAACAGCTGCAGC |
| | | AGAACAACATCGTTTACTTCGATCTGGACAAGTA CGATATCCGTTCTGACTTCGCTCAAATGCTGGAT GCACATGCAAACTTCCTGCGTAGCAACCCGTCTT ACAAAGTCACCGTAGAAGGTCACGCGGACGAAC GTGGTACTCCGGAATACAACATCTCCCTGGGTGA ACGTCGTGCGAACGCCGTTAAGATGTACCTGCAG GGTAAAGGCGTTTCTGCAGACCAGATCTCCATCG TTTCTTACGGTAAAGAAAAACCTGCAGTACTGGG TCATGACGAAGCGGCATACTCCGCGGTACTGGTT TACTAA |
| 49 | Lpp | CSSNAKIDQLSSDVQTLNAKVDQLSNDVNAMRSDV OAAKDDAARANORLDNMATKYRK* |
| 50 | OmpA | APKDNTWYTGAKLGWSQYHDTGFINNNGPTHENQ LGAGAFGGYQVNPYVGFEMGYDWLGRMPYKGSV ENGAYKAQGVQLTAKLGYPITDDLDIYTRLGGMV WRADTKSNVYGKNHDTGVSPVFAGGVEYAITPEIA TRLEYQWTNNIGDAHTIGTRPDNGMLSLGVSYRFG QGEAAPVVAPAPAPAPEVQTKHFTLKSDVLFNFNK ATLKPEGQAALDQLYSQLSNLDPKDGSVVVLGYTD RIGSDAYNQGLSERRAQSVVDYLISKGIPADKISAR GMGESNPVTGNTCDNVKQRAALIDCLAPDRRVEIE VKGIKDVVTQPQA |
| 51 | TolA | MSKATEQNDKLKRAIIISAVLHVILFAALIWSSFDEN IEASAGGGGGSSIDAVMVDSGAVVEQYKRMQSQES SAKRSDEQRKMKEQQAAEELREKQAAEQERLKQL EKERLAAQEQKKQAEEAAKQAELKQKQAEEAAAK AAADAKAKAEADAKAAEEAAKKAAADAKKKAEA EAAKAAAEAQKKAEAAAAALKKKAEAAEAAAAE ARKKAATEAAEKAKAEAEKKAAAEKAAADKKAA AEKAAADKKAAEKAAAEKAAADKKAAAEKAAAD KKAAAAKAAAEKAAAAKAAAEADDIFGELSSGKN APKTGGGAKGNNASPAGSGNTKNNGASGADINNY AGQIKSAIESKFYDASSYAGKTCTLRIKLAPDGMLL DIKPEGGDPALCQAALAAAKLAKIPKPPSQAVYEVF KNAPLDFKP |
| 52 | Pal 19-43 | GTGMDANGGNGNMSSEEQARLQMQQ |
| 53 | Pal144-147 | SKNR |
| 54 | Structural gene of ybiS | GTAACTTATCCTCTGCCAACCGACGGGAGTCGCC TGGTTGGTCAGAATCAGGTGATCACCATTCCTGA AGGTAACACTCAGCCGCTGGAGTATTTTGCCGCC GAGTACCAGATGGGGCTTTCCAATATGATGGAAG CGAACCCGGGTGTGGATACCTTCCTGCCGAAAGG CGGTACTGTACTGAACATTCCGCAGCAGCTGATC |
| | | CTGCCGGATACCGTTCATGAAGGCATCGTCATTA ACAGTGCTGAGATGCGTCTTTATTACTATCCGAA AGGGACCAACACCGTTATCGTGCTGCCGATCGGC ATTGGTCAGTTAGGCAAAGATACGCCTATCAACT GGACCACCAAAGTTGAGCGTAAAAAAGCAGGCC CGACCTGGACGCCGACCGCCAAAATGCACGCAG AGTACCGCGCTGCGGGCGAACCGCTTCCGGCTGT CGTTCCGGCAGGTCCGGATAACCCGATGGGGCTG TATGCACTCTATATCGGTCGCCTGTATGCTATCCA TGGCACCAACGCCAACTTCGGTATCGGCCTGCGT GTAAGTCATGGTTGTGTGCGTCTGCGTAACGAAG ACATCAAATTCCTGTTCGAGAAAGTACCGGTCGG TACCCGCGTACAGTTTATTGATGAGCCGGTAAAA GCGACCACCGAGCCAGACGGCAGCCGTTATATTG AAGTCCATAACCCGCTGTCTACCACCGAAGCCCA GTTTGAAGGTCAGGAAATTGTGCCAATTACCCTG ACGAAGAGCGTGCAGACAGTGACCGGTCAGCCA GATGTTGACCAGGTTGTTCTTGATGAAGCGATTA AAAACCGCTCCGGGATGCCGGTTCGTCTGAATTA A |
| 55 | Nucleotide sequence of ybiS signal | ATGAATATGAAATTGAAAACATTATTCGCAGCGG CCTTCGCTGTTGTCGGCTTTTGCAGTACCGCCTCT GCG |
| 56 | Structural gene of ycfS | CTACCGGCAAAAGCCAACACCTGGCCGCTGCCGC CAGCGGGCAGTCGTCTGGTTGGCGAAAACAAATT TCATGTGGTGGAAAATGACGGTGGTTCTCTGGAA GCCATCGCCAAAAAATACAACGTCGGCTTTCTCG CTCTGTTACAGGCTAACCCCGGCGTTGATCCTTAC GTACCGCGCGCGGGCAGCGTGTTAACGATCCCGT TGCAAACCCTACTTCCAGATGCGCCGCGCGAAGG CATTGTGATCAACATTGCGGAGCTGCGTCTCTATT ACTACCCGCCGGGTAAAAATTCGGTAACCGTGTA TCCAATAGGTATTGGTCAGTTAGGTGGTGACACG CTGACACCGACAATGGTGACCACCGTTTCAGACA AACGTGCAAACCCAACCTGGACGCCAACGGCAA ACATCCGCGCCCGTTATAAAGCACAGGGAATTGA GTTGCCTGCGGTAGTGCCGGCTGGACTGGATAAC CCAATGGGCCATCATGCGATTCGTCTGGCGGCCT ATGGCGGCGTTTATTTGCTTCATGGTACGAACGC CGATTTCGGCATTGGCATGCGGGTAAGTTCTGGC TGTATTCGTCTGCGGGATGACGATATCAAAACAC TCTTTAGCCAGGTCACCCCAGGCACCAAAGTGAA TATCATCAACACTCCGATAAAAGTCTCTGCCGAA |
| | | CCAAACGGTGCGCGTCTGGTTGAAGTACATCAGC CGCTGTCAGAGAAGATTGATGACGATCCGCAGCT GCTGCCAATTACGCTGAATAGCGCAATGCAATCA TTTAAAGATGCAGCACAAACTGACGCTGAAGTGA TGCAACATGTGATGGATGTCCGTTCCGGGATGCC GGTGGATGTCCGCCGTCATCAAGTGAGCCCACAA ACGCTGTAA |
| 57 | Nucleotide sequence of ycfS signal | GTGATGATCAAAACGCGTTTTTCTCGCTGGCTAA CGTTTTTTACGTTCGCCGCTGCCGTGGCGCTGGCG |
| 58 | Structural gene of erfK | GTAACTTATCCATTACCTCCAGAGGGTAGCCGTT TAGTGGGGCAGTCGTTTACTGTAACTGTTCCTGAT CACAATACCCAGCCGCTGGAGACTTTTGCCGCAC AATACGGGCAAGGGTTAAGTAACATGCTGGAAG CGAACCCGGGCGCTGATGTTTTTTTGCCGAAGTC TGGCTCGCAACTCACCATTCCGCAGCAACTGATT TTGCCCGACACTGTTCGTAAAGGGATTGTTGTTA ACGTCGCTGAGATGCGTCTTTATTACTACCCACC AGACAGTAATACTGTGGAAGTCTTTCCTATTGGT ATCGGCCAGGCTGGGCGAGAAACCCCGCGTAACT GGGTGACTACCGTTGAACGTAAACAAGAAGCTCC AACCTGGACGCCAACGCCGAACACTCGGCGCGA ATATGCGAAACGAGGGGAGAGTTTGCCCGCATTT GTTCCTGCGGGCCCCGATAATCCCATGGGGCTGT ACGCGATTTATATTGGCAGGTTGTATGCCATCCA TGGTACCAATGCCAATTTTGGTATTGGGCTCCGG GTAAGTCAGGGCTGTATTCGTCTGCGCAATGACG ATATCAAATATCTGTTTGATAATGTTCCTGTTGGG ACGCGTGTGCAAATTATCGACCAGCCAGTAAAAT ACACCACTGAACCAGATGGCTCGAACTGGCTGGA AGTTCATGAGCCATTGTCGCGCAATCGTGCAGAA TATGAGTCTGACCGAAAAGTGCCATTGCCGGTAA CCCCATCTTTGCGGGCGTTTATCAACGGGCAAGA AGTTGATGTAAATCGCGCAAATGCTGCGTTGCAA CGTCGATCGGGAATGCCTGTGCAAATTAGTTCTG GTTCAAGACAGATGTTTTAA |
| 59 | Nucleotide sequence of erfK signal | ATGCGTCGTGTAAATATTCTTTGCTCATTTGCTCT GCTTTTTGCCAGCCATACTAGCCTGGCG |
| 60 | Lpp signal peptide | MKATKLVLGAVILGSTLLAG |
| 61 | OmpA signal peptide | MKKTAIAIAVALAGFATVAQA |
| 62 | Structural gene of bamA | GCTGAAGGGTTCGTAGTGAAAGATATTCATTTCG AAGGCCTTCAGCGTGTCGCCGTTGGTGCGGCCCT CCTCAGTATGCCGGTGCGCACAGGCGACACGGTT AATGATGAAGATATCAGTAATACCATTCGCGCTC |
| | | TGTTTGCTACCGGCAACTTTGAGGATGTTCGCGTC CTTCGTGATGGTGATACCCTTCTGGTTCAGGTAA AAGAACGTCCGACCATTGCCAGCATTACTTTCTC CGGTAACAAATCGGTGAAAGATGACATGCTGAA GCAAAACCTCGAGGCTTCTGGTGTGCGTGTGGGC GAATCCCTCGATCGCACCACCATTGCCGATATCG AGAAAGGTCTGGAAGACTTCTACTACAGCGTCGG TAAATATAGCGCCAGCGTAAAAGCTGTCGTGACC CCGCTGCCGCGCAACCGTGTTGACCTAAAACTGG TGTTCCAGGAAGGTGTGTCAGCTGAAATCCAGCA AATTAACATTGTTGGTAACCATGCTTTCACCACC GACGAACTGATCTCTCATTTCCAACTGCGTGACG AAGTGCCGTGGTGGAACGTGGTAGGCGATCGTAA ATACCAGAAACAGAAACTGGCGGGCGACCTTGA AACCCTGCGCAGCTACTATCTGGATCGCGGTTAT GCCCGTTTCAACATCGACTCTACCCAGGTCAGTC TGACGCCAGATAAAAAAGGTATTTACGTCACGGT GAACATCACCGAAGGCGATCAGTACAAGCTTTCT GGCGTTGAAGTGAGCGGCAACCTTGCCGGGCACT CCGCTGAAATTGAGCAGCTGACTAAGATCGAGCC GGGTGAGCTGTATAACGGCACCAAAGTGACCAA GATGGAAGATGACATCAAAAAGCTTCTCGGTCGC TATGGTTATGCCTATCCGCGCGTACAGTCGATGC CCGAAATTAACGATGCCGACAAAACCGTTAAATT ACGTGTGAACGTTGATGCGGGTAACCGTTTCTAC GTGCGTAAGATCCGTTTTGAAGGTAACGATACCT CGAAAGATGCCGTCCTGCGTCGCGAAATGCGTCA GATGGAAGGTGCATGGCTGGGGAGCGATCTGGTC GATCAGGGTAAGGAGCGTCTGAATCGTCTGGGCT TCTTTGAAACTGTCGATACCGATACCCAACGTGT TCCGGGTAGCCCGGACCAGGTTGATGTCGTCTAC AAGGTAAAAGAGCGCAACACCGGTAGCTTCAAC TTTGGTATTGGTTACGGTACTGAAAGTGGCGTGA GCTTCCAGGCTGGTGTGCAGCAGGATAACTGGTT AGGTACAGGTTATGCTGTTGGTATCAACGGGACC AAAAACGATTACCAGACCTATGCTGAACTGTCGG TAACCAACCCGTACTTCACCGTAGATGGCGTAAG CCTCGGTGGTCGTCTCTTCTATAATGACTTCCAGG CAGATGACGCCGACCTGTCCGACTATACCAACAA GAGTTATGGTACAGACGTGACGTTGGGCTTCCCG ATTAACGAATATAACTCGCTGCGTGCAGGTCTGG GTTATGTACATAACTCCCTGTCCAACATGCAGCC TCAGGTTGCGATGTGGCGTTATCTGTACTCTATGG |
| | | GTGAACATCCGAGCACCTCTGATCAGGATAACAG CTTCAAAACGGACGACTTCACGTTCAACTATGGT TGGACCTATAACAAGCTTGACCGTGGTTACTTCC CGACAGATGGTTCACGTGTCAACCTGACCGGTAA AGTGACCATTCCTGGATCGGATAACGAATACTAC AAAGTGACGTTAGACACGGCGACTTATGTGCCGA TCGATGACGATCACAAATGGGTTGTTCTGGGGCG TACCCGCTGGGGTTATGGTGATGGTTTAGGCGGC AAAGAGATGCCGTTCTACGAGAACTTCTATGCCG GTGGTTCCAGCACCGTGCGTGGCTTCCAGTCCAA TACCATTGGTCCGAAAGCAGTTTACTTCCCGCAT CAGGCCAGTAATTATGATCCGGACTATGATTACG AATGTGCGACTCAGGACGGCGCGAAAGACCTGT GTAAATCGGATGATGCTGTAGGCGGTAACGCCAT GGCGGTTGCCAGCCTCGAGTTCATCACCCCGACG CCGTTTATTAGCGATAAGTATGCTAACTCGGTTC GTACTTCCTTCTTCTGGGATATGGGTACCGTTTGG GATACAAACTGGGATTCCAGCCAATATTCTGGAT ATCCGGACTATAGTGATCCAAGCAATATCCGTAT GTCTGCGGGTATCGCATTACAATGGATGTCCCCA TTGGGGCCGTTGGTGTTCTCCTACGCCCAGCCGTT CAAAAAGTACGATGGAGACAAGGCAGAACAGTT CCAGTTTAACATCGGTAAAACCTGGTAA |
| 63 | Nucleotide sequence of bamA signal | ATGGCGATGAAAAAGTTGCTCATAGCGTCGCTGC TGTTTAGCAGCGCCACCGTATACGGT |
| 64 | Structural gene of lolA | GATGCCGCAAGCGATCTGAAAAGCCGCCTGGATA AAGTCAGCAGCTTCCACGCCAGCTTCACACAAAA AGTGACTGACGGTAGCGGCGCGGCGGTGCAGGA AGGTCAGGGCGATCTGTGGGTGAAACGTCCAAAC TTATTCAACTGGCATATGACACAACCTGATGAAA GCATTCTGGTTTCTGACGGTAAAACACTGTGGTT CTATAACCCGTTCGTTGAGCAAGCTACGGCAACC TGGCTGAAAGATGCCACCGGTAATACGCCGTTTA TGCTGATTGCCCGCAACCAGTCCAGCGACTGGCA GCAGTACAATATCAAACAGAATGGCGATGACTTT GTCCTGACGCCGAAAGCCAGCAATGGCAATCTGA AGCAGTTCACCATTAACGTGGGACGTGATGGCAC AATCCATCAGTTTAGCGCGGTGGAGCAGGACGAT CAGCGCAGCAGTTATCAACTGAAATCCCAGCAAA ATGGGGCTGTGGATGCAGCGAAATTTACCTTCAC CCCGCCGCAAGGCGTCACGGTAGATGATCAACGT AAGTAG |
| 65 | Nucleotide sequence of lolA signal | ATGAAAAAAATTGCCATCACCTGTGCATTACTCT CAAGCTTAGTAGCAAGCAGCGTTTGGGCT |
| 66 | Structural gene of lolB | TGTTCCGTTACCACGCCCAAAGGTCCTGGCAAAA GCCCGGATTCGCCACAATGGCGTCAGCATCAGCA AGACGTGCGCAATCTTAATCAGTATCAGACTCGC GGCGCGTTCGCTTATATTTCTGACCAACAAAAAG TGTACGCCCGCTTTTTCTGGCAGCAAACCGGCCA GGATCGCTACCGTCTGCTGCTCACTAACCCATTG GGCAGCACGGAACTGGAGCTGAATGCTCAACCG GGTAACGTGCAGTTAGTCGACAATAAAGGTCAGC GTTATACCGCCGATGACGCCGAAGAGATGATTGG CAAATTGACCGGAATGCCAATTCCGCTCAACAGC TTGCGCCAGTGGATTTTAGGTTTACCGGGTGATG CAACCGACTACAAACTGGACGACCAGTACCGCCT GAGCGAAATTACCTACAGCCAGAATGGCAAAAA CTGGAAGGTTGTTTATGGTGGTTATGACACCAAA ACGCAACCTGCGATGCCAGCCAATATGGAACTCA CCGACGGTGGTCAACGCATCAAGTTAAAAATGGA TAACTGGATAGTGAAATAA |
| 67 | Nucleotide sequence of lolB signal | ATGCCCCTGCCCGATTTTCGTCTTATCCGCCTGCT ACCGCTGGCTGCTCTTGTGCTCACTGCC |
| 68 | Structural gene of lolC | ATGTACCAACCTGTCGCTCTATTTATTGGCCTGCG TTACATGCGTGGGCGTGCAGCGGATCGCTTCGGT CGTTTCGTCTCCTGGCTTTCTACCATCGGCATTAC CCTCGGGGTGATGGCGCTGGTCACAGTATTGTCA GTGATGAACGGCTTTGAGCGCGAGCTGCAAAACA ACATCCTTGGCCTGATGCCACAGGCAATTCTCTCT TCTGAGCATGGCTCTCTTAACCCGCAGCAACTCC CAGAAACGGCAGTCAAACTGGACGGCGTTAATC GCGTCGCACCTATTACTACCGGTGATGTGGTACT GCAAAGCGCGCGCAGCGTGGCGGTCGGGGTGAT GCTCGGTATCGACCCGGCGCAAAAAGATCCACTT ACACCGTATCTGGTCAATGTGAAACAAACTGACC TCGAGCCGGGGAAATATAATGTCATCCTCGGCGA ACAACTTGCCTCACAGCTAGGCGTTAATCGCGGT GATCAAATCCGCGTGATGGTACCATCTGCCAGCC AGTTCACGCCGATGGGGCGTATTCCAAGCCAGCG CCTGTTCAATGTGATTGGCACTTTCGCCGCCAAC AGTGAAGTCGATGGCTATGAAATGCTGGTGAATA TTGAGGATGCCTCGCGTCTGATGCGTTATCCGGC AGGCAATATTACCGGCTGGCGTTTGTGGCTGGAT GAGCCGCTGAAAGTCGACTCATTAAGTCAGCAAA AACTGCCTGAAGGCAGCAAATGGCAGGACTGGC |
| | | GTGATCGTAAAGGCGAGTTGTTCCAGGCCGTACG CATGGAAAAAAATATGATGGGTTTACTGCTGAGC CTGATTGTCGCCGTTGCGGCGTTTAACATTATTAC CTCACTAGGGCTGATGGTAATGGAGAAGCAGGG CGAAGTAGCGATCCTGCAAACGCAAGGCTTAACT CCGCGACAAATCATGATGGTCTTTATGGTGCAAG GGGCCAGCGCCGGGATTATCGGTGCGATCCTCGG AGCGGCGCTTGGCGCCCTGCTTGCCAGCCAGTTA AATAATCTGATGCCGATAATCGGCGTCCTGCTTG ATGGCGCGGCGCTGCCGGTGGCTATCGAACCTTT ACAGGTCATTGTTATTGCGCTGGTGGCGATGGCT ATCGCGCTGCTGTCTACGCTTTACCCTTCATGGCG CGCTGCCGCCACTCAACCCGCTGAGGCTTTACGT TATGAATAA |
| 69 | Structural gene of lolD | ATGAATAAGATCCTGTTGCAATGCGACAACCTGT GCAAACGCTATCAGGAAGGCAGTGTGCAAACCG ATGTGCTGCACAATGTCAGTTTCAGCGTCGGCGA AGGTGAAATGATGGCGATCGTCGGTAGCTCTGGT TCCGGTAAAAGTACCTTGCTGCACCTGCTGGGCG GGCTGGATACGCCAACCTCCGGCGATGTGATTTT TAATGGTCAGCCAATGAGCAAACTGTCGTCGGCA GCGAAAGCTGAACTGCGCAACCAGAAGCTGGGC TTTATTTATCAGTTTCACCACCTGCTGCCGGATTT TACCGCCCTGGAAAACGTGGCTATGCCGCTGCTG ATTGGCAAGAAAAAGCCCGCTGAAATCAACAGC CGTGCACTTGAGATGTTAAAAGCGGTGGGGCTGG ATCATCGTGCGAATCACCGCCCATCTGAACTTTCT GGCGGCGAACGCCAGCGTGTGGCGATTGCCCGTG CGCTGGTGAATAACCCGCGCCTGGTACTGGCGGA TGAACCTACCGGTAACCTCGATGCGCGTAACGCC GACAGCATCTTCCAGTTGCTTGGGGAATTGAATC GCTTGCAGGGCACCGCCTTCCTGGTGGTTACTCA CGACCTGCAACTGGCGAAACGTATGAGCCGCCAG TTAGAAATGCGTGATGGTCGTCTGACGGCGGAAC TGAGCCTGATGGGGGCGGAGTAA |
| 70 | Structural gene of lolE | ATGGCGATGCCTTTATCGTTATTAATTGGCCTGCG TTTTAGTCGCGGACGGCGGCGCGGCGGCATGGTG TCGCTGATCTCCGTTATTTCTACCATTGGCATTGC CCTCGGCGTGGCGGTATTGATCGTCGGCTTAAGC GCGATGAACGGCTTTGAACGCGAACTGAACAACC GTATTCTGGCGGTGGTGCCGCATGGTGAAATCGA GGCGGTGGATCAGCCGTGGACTAACTGGCAGGA AGCACTGGATCACGTGCAAAAAGTGCCGGGTATT |
| | | GCCGCTGCCGCGCCGTATATCAATTTCACCGGGC TGGTGGAAAGTGGCGCGAATCTTCGCGCAATCCA GGTGAAGGGCGTTAACCCGCAACAGGAACAGCG TCTGAGCGCATTACCCTCGTTTGTCCAGGGCGAT GCGTGGCGCAATTTTAAAGCGGGCGAACAGCAA ATTATCATCGGCAAAGGCGTGGCGGATGCGCTGA AAGTGAAGCAGGGGGATTGGGTGTCGATTATGAT CCCCAACTCGAATCCCGAGCATAAACTGATGCAG CCAAAACGTGTGCGTTTGCACGTTGCCGGTATTTT GCAGTTGAGTGGTCAACTCGATCACAGTTTTGCC ATGATCCCGCTGGCGGATGCCCAACAATATCTTG ATATGGGTTCCAGCGTGTCAGGTATTGCCCTTAA AATGACGGATGTTTTCAACGCCAATAAGCTGGTA CGCGATGCGGGTGAAGTGACCAACAGCTATGTTT ATATTAAAAGCTGGATTGGTACTTACGGCTATAT GTATCGCGATATCCAAATGATCCGCGCCATTATG TATCTGGCGATGGTACTGGTGATTGGCGTGGCCT GTTTCAACATCGTCTCCACCTTAGTGATGGCGGT GAAAGACAAGAGTGGCGATATCGCAGTATTAAG AACGCTGGGGGCGAAAGATGGTTTAATTCGCGCC ATCTTTGTCTGGTATGGATTGCTGGCAGGGCTGTT CGGCAGCCTGTGTGGTGTGATTATCGGCGTAGTG GTTTCACTGCAACTTACCCCGATTATTGAGTGGAT TGAAAAGTTGATCGGTCATCAGTTCCTCTCCAGC GATATCTATTTTATTGATTTCCTGCCATCGGAATT GCACTGGCTGGACGTCTTCTACGTACTGGTCACA GCATTGTTGCTGAGTCTTTTGGCAAGTTGGTATCC GGCGCGGCGCGCCAGTAATATTGACCCTGCGCGA GTCCTTAGCGGCCAGTAA |
| 71 | Structural gene of lptE | TGTGGCTGGCATCTGCGTGATACCACGCAGGTTC CTTCCACTATGAAGGTCATGATCCTGGACTCAGG CGATCCGAACGGGCCATTAAGCCGTGCGGTGCGT AACCAGTTACGTCTGAATGGTGTCGAGTTGCTTG ATAAAGAAACCACGCGTAAGGACGTTCCATCCTT GCGTTTGGGTAAAGTGAGCATCGCGAAAGATACC GCATCGGTATTCCGTAACGGTCAAACAGCAGAGT ATCAGATGATCATGACGGTTAATGCGACCGTGTT GATCCCCGGCCGTGATATCTACCCGATTAGCGCC AAAGTCTTCCGTTCGTTCTTCGATAACCCGCAAAT GGCGTTAGCGAAAGATAACGAACAAGACATGAT CGTAAAAGAGATGTACGACCGTGCTGCCGAACA GCTGATTCGTAAGCTGCCAAGCATCCGTGCTGCG GATATTCGTTCCGACGAAGAACAGACGTCGACCA |
| | | CAACGGATACTCCGGCAACGCCTGCACGCGTCTC CACCACGCTGGGTAACTGA |
| 72 | Nucleotide sequence of lptE signal | GTGCGATATCTGGCAACATTGTTGTTATCTCTGGC GGTGTTAATCACCGCCGGG |
| 73 | Structural gene of lptD | GCCGACCTCGCCTCACAGTGCATGTTGGGCGTGC CAAGCTATGACCGTCCTCTGGTACAGGGCGATAC CAATGACTTACCCGTGACTATCAATGCTGACCAC GCGAAAGGGGACTACCCGGATGACGCCGTGTTTA CTGGCAGCGTGGATATCATGCAGGGTAACAGCCG TCTGCAGGCCGACGAAGTGCAGCTCCATCAAAAA GAGGCACCAGGACAACCGGAGCCGGTACGTACC GTTGATGCGCTCGGTAATGTCCATTACGACGATA ACCAGGTGATCCTCAAAGGGCCGAAAGGCTGGG CGAATCTGAACACCAAAGATACCAACGTCTGGGA AGGTGATTACCAGATGGTGGGTCGCCAGGGTCGC GGTAAAGCGGACCTGATGAAACAACGTGGCGAA AACCGCTATACCATTCTGGATAACGGTAGCTTTA CCTCCTGTCTGCCGGGTTCTGACACCTGGAGCGT GGTAGGTAGCGAAATTATTCATGACCGCGAAGAA CAAGTTGCGGAGATCTGGAACGCCCGCTTTAAGG TGGGTCCGGTACCGATCTTTTATAGCCCCTATTTG CAGTTGCCGGTGGGTGACAAACGTCGCTCTGGTT TCTTGATCCCGAACGCCAAGTACACCACCACCAA CTACTTTGAGTTCTACCTGCCATATTACTGGAACA TCGCGCCAAATATGGATGCCACCATCACGCCGCA TTATATGCATCGTCGTGGCAACATCATGTGGGAG AACGAATTCCGCTACCTCTCCCAGGCGGGCGCTG GCTTGATGGAACTGGACTATCTGCCTTCAGATAA AGTCTATGAAGATGAACACCCGAACGATGACAGT TCACGTCGTTGGTTATTCTACTGGAACCACTCCGG GGTCATGGATCAGGTGTGGCGTTTCAACGTCGAC TACACCAAGGTCAGCGATCCTAGCTACTTCAATG ATTTCGATAACAAGTACGGTTCCAGTACTGACGG CTACGCAACGCAAAAATTCAGCGTTGGCTATGCG GTGCAAAACTTCAATGCCACCGTTTCAACCAAGC AGTTCCAGGTTTTCAGCGAACAGAACACCAGTAG CTACTCGGCAGAGCCGCAGTTAGACGTTAATTAC TACCAGAATGATGTTGGTCCGTTTGATACGCGTA TTTACGGCCAGGCAGTGCACTTTGTTAACACCAG AGACGACATGCCTGAAGCAACCCGTGTTCACCTG GAACCGACCATCAATTTGCCGCTCTCTAATAACT GGGGCAGCATCAATACCGAAGCGAAGTTGCTGG CAACCCATTATCAGCAAACCAATCTTGACTGGTA |
| | | TAACTCCAGAAACACGACCAAGCTGGACGAATCC GTTAACCGCGTAATGCCGCAATTCAAAGTTGACG GCAAAATGGTCTTTGAACGCGATATGGAAATGCT GGCTCCGGGTTATACCCAAACGCTGGAACCGCGC GCGCAGTATTTGTACGTGCCGTATCGCGATCAGA GCGACATCTATAACTACGACTCGTCTCTGCTGCA ATCTGACTACTCTGGCCTGTTCCGGGACCGGACT TACGGCGGTCTTGACCGTATTGCCTCCGCTAACC AGGTGACGACCGGTGTCACATCTCGCATATATGA TGATGCTGCCGTTGAACGTTTTAATATTTCCGTTG GTCAAATCTACTATTTCACGGAGTCTCGCACTGG CGATGACAACATAACATGGGAGAATGACGACAA AACGGGTTCACTGGTGTGGGCAGGCGATACTTAC TGGCGTATCTCCGAGCGTTGGGGATTGCGTGGCG GGATTCAGTACGATACACGTCTGGATAACGTAGC GACCAGTAACTCCAGCATTGAATACCGTCGGGAT GAAGACCGTCTGGTACAGCTGAATTACCGTTACG CCAGCCCGGAATATATTCAGGCTACGCTGCCTAA GTACTATTCCACTGCTGAGCAATATAAGAATGGT ATTTCGCAGGTAGGTGCTGTCGCCAGCTGGCCAA TTGCCGATCGTTGGTCCATTGTTGGGGCCTACTAC TACGACACCAATGCTAACAAGCAAGCCGACTCTA TGTTAGGTGTGCAATACAGCTCCTGCTGCTATGC AATTCGCGTCGGTTACGAGCGGAAGCTGAACGGT TGGGATAACGATAAACAACATGCGGTATATGACA ACGCAATCGGCTTTAACATCGAACTTCGCGGCCT GAGCTCCAACTACGGTCTGGGTACGCAAGAGATG CTGCGTTCGAACATTCTGCCGTATCAAAACACTTT GTGA |
| 74 | Nucleotide sequence of lptD signal | ATGAAAAAACGTATCCCCACTCTCCTGGCCACCA TGATTGCCACCGCCCTTTATAGTCAACAGGGACT GGCA |
| 75 | Structural gene of lptA | GTAACCGGAGACACTGATCAGCCGATCCACATTG AATCGGACCAGCAATCTCTTGATATGCAAGGCAA CGTGGTTACCTTTACCGGTAATGTCATCGTCACCC AGGGCACCATCAAAATTAATGCCGACAAAGTGGT CGTTACCCGTCCGGGCGGCGAACAAGGTAAAGA AGTGATTGACGGCTACGGTAAACCGGCAACGTTC TACCAGATGCAGGACAACGGTAAACCCGTTGAA GGTCACGCTTCCCAGATGCACTACGAACTGGCAA AAGATTTTGTCGTTCTGACGGGTAATGCTTATCTG CAGCAGGTCGATAGCAACATTAAGGGCGATAAG ATCACTTACCTGGTGAAAGAGCAGAAAATGCAG |
| | | GCTTTCAGCGACAAAGGCAAGCGCGTAACAACC GTTCTGGTGCCGTCGCAGCTGCAGGACAAAAACA ACAAAGGCCAGACCCCGGCACAGAAGAAGGGTA ATTAA |
| 76 | Nucleotide sequence of lptA signal | ATGAAATTCAAAACAAACAAACTCAGCCTTAATC TTGTGCTTGCCAGCTCACTTCTGGCCGCCAGCATT CCGGCATTTGCC |
| 77 | Structural gene of lptB | ATGGCAACATTAACTGCAAAGAACCTTGCAAAAG CCTATAAAGGCCGTCGCGTGGTAGAAGACGTCAG CCTGACCGTCAACTCCGGGGAAATTGTCGGTCTG CTGGGGCCAAACGGTGCCGGTAAGACCACCACTT TCTACATGGTTGTAGGCATTGTGCCGCGCGATGC GGGCAACATCATTATTGATGATGACGATATCAGT CTGCTGCCTCTGCATGCACGCGCGCGCCGCGGTA TCGGCTATCTGCCACAGGAAGCCTCCATTTTCCGT CGCCTCAGCGTTTACGATAACCTGATGGCGGTAC TGCAAATTCGTGACGACTTGTCTGCTGAACAACG TGAAGACCGCGCGAACGAGCTGATGGAAGAGTT TCACATTGAGCACCTGCGTGACAGCATGGGGCAG TCACTCTCCGGGGGTGAACGTCGCCGTGTAGAAA TTGCCCGCGCACTGGCTGCGAATCCGAAATTTAT TCTGCTCGACGAACCGTTTGCCGGGGTTGACCCG ATCTCGGTTATCGACATTAAACGCATCATTGAGC ACCTGCGCGACAGCGGCCTGGGCGTGCTGATCAC TGACCACAACGTGCGTGAAACACTGGCGGTTTGT GAACGCGCTTATATCGTCAGTCAGGGGCATTTGA TCGCCCACGGCACGCCTACAGAAATCTTACAAGA CGAACACGTTAAGCGTGTATACCTTGGGGAAGAC TTCAGACTCTGA |
| 78 | Structural gene of lptF | GTGATAATCATAAGATATCTGGTGCGGGAGACGC TCAAAAGCCAGCTGGCGATACTCTTCATCTTGCTT TTGATCTTCTTCTGTCAAAAGTTAGTGAGGATCCT CGGCGCAGCGGTTGACGGCGATATTCCGGCGAAT CTGGTGCTCTCCCTTCTCGGGTTGGGCGTGCCGG AAATGGCGCAGCTTATCCTGCCATTAAGCCTGTT CCTCGGGCTGCTGATGACGCTGGGCAAACTGTAT ACCGAAAGTGAAATTACGGTAATGCATGCCTGCG GCCTGAGCAAAGCGGTTCTGGTGAAAGCGGCAAT GATCCTTGCGGTATTCACGGCAATCGTAGCGGCG GTTAACGTGATGTGGGCGGGACCGTGGTCATCGC GTCATCAGGATGAAGTGTTAGCAGAAGCGAAAG CGAACCCTGGCATGGCGGCGCTGGCGCAAGGGC AATTCCAGCAAGCGACTAATGGCAGCTCGGTGCT |
| | | GTTCATCGAAAGCGTTGACGGCAGCGATTTCAAA GATGTGTTCCTCGCGCAAATTCGACCAAAAGGTA ATGCACGTCCTTCTGTGGTGGTGGCCGATTCCGG ACATTTAACCCAGCTGCGCGACGGCTCCCAGGTC GTCACTCTCAACCAGGGAACGCGCTTCGAAGGCA CTGCATTGTTACGTGATTTCCGCATTACGGACTTC CAGGATTATCAGGCGATCATTGGTCACCAGGCGG TGGCGCTCGACCCGAACGATACCGACCAGATGGA CATGCGCACATTGTGGAACACTGACACCGATCGT GCTCGCGCAGAACTGAACTGGCGTATCACGTTGG TATTCACCGTGTTTATGATGGCACTTATGGTCGTA CCGCTGAGCGTGGTTAACCCACGTCAGGGACGCG TACTGTCGATGCTGCCAGCCATGCTGCTGTATCTA CTTTTCTTCCTGATCCAGACCTCCCTGAAATCGAA CGGCGGTAAAGGTAAGCTGGACCCGACGCTGTG GATGTGGACCGTTAACCTGATTTATCTGGCTTTAG CGATTGTTCTCAACCTTTGGGACACCGTGCCGGT CCGCCGCCTGCGCGCCAGTTTTTCGCGTAAAGGA GCGGTGTG |
| 79 | Structural gene of lptG | ATGCAACCTTTTGGCGTACTTGACCGCTATATCG GTAAAACTATTTTCACCACCATCATGATGACACT GTTCATGCTGGTGTCGCTGTCGGGCATTATCAAG TTTGTCGATCAGCTGAAAAAAGCCGGGCAGGGG AGTTACGACGCGTTAGGCGCAGGAATGTATACCT TGCTGAGCGTGCCGAAAGATGTGCAGATCTTCTT CCCGATGGCGGCTCTGCTTGGGGCGTTGCTTGGT CTTGGGATGCTGGCGCAGCGCAGCGAACTGGTGG TGATGCAGGCTTCTGGTTTTACCCGTATGCAGGT GGCGCTGTCGGTGATGAAAACCGCCATTCCGCTG GTCTTGCTGACGATGGCGATTGGCGAATGGGTCG CGCCGCAGGGCGAGCAGATGGCGCGTAACTACC GTGCGCAGGCGATGTACGGCGGCTCGTTGCTCTC TACCCAGCAAGGCTTATGGGCGAAAGATGGCAA CAACTTCGTCTACATTGAGCGGGTTAAAGGTGAC GAAGAGTTAGGTGGCATCAGCATTTATGCCTTTA ACGAGAATCGTCGTCTGCAATCCGTACGCTATGC CGCTACTGCGAAGTTTGACCCGGAACATAAAGTC TGGCGTCTGTCGCAGGTTGATGAATCTGATCTGA CCAATCCGAAACAGATTACCGGTTCGCAGACGGT GAGCGGCACCTGGAAAACCAACCTCACGCCGGA CAAACTGGGCGTGGTGGCGCTGGACCCGGATGCA CTCTCTATCAGCGGTTTGCACAACTATGTGAAGT ATCTGAAGTCGAGCGGTCAGGATGCCGGACGTTA |
| | | TCAGCTCAACATGTGGAGCAAAATCTTCCAGCCG CTATCTGTGGCGGTGATGATGCTGATGGCGCTGT CGTTCATCTTTGGCCCACTGCGTAGCGTACCGAT GGGCGTGCGTGTGGTCACCGGTATCAGTTTCGGT TTTGTCTTCTACGTACTGGACCAGATCTTCGGCCC GCTGACGTTGGTTTATGGCATCCCGCCGATCATC GGCGCACTGTTGCCAAGCGCCAGCTTCTTCTTAA TCAGCCTGTGGCTGTTAATGAGAAAATCGTAA |
| 80 | Structural gene of lpoA | TGTGGCACCCATACTCCCGATCAGTCCACTGCTT ATATGCAGGGCACGGCGCAGGCTGATTCTGCCTT TTATCTTCAGCAGATGCAGCAAAGCTCTGATGAT ACCAGGATCAACTGGCAATTACTCGCCATTCGTG CACTGGTGAAAGAAGGTAAAACCGGGCAGGCGG TTGAGTTGTTTAACCAACTACCGCAAGAACTGAA CGATGCTCAGCGTCGCGAGAAAACACTGCTGGCG GTAGAGATTAAACTGGCGCAGAAAGATTTTGCTG GCGCGCAAAACTTGCTGGCGAAAATCACACCTGC CGATTTAGAACAAAACCAGCAAGCGCGTTACTGG CAGGCAAAAATCGATGCCAGCCAGGGGCGTCCTT CCATTGATTTACTGCGCGCGTTAATTGCTCAGGA ACCGCTGCTTGGCGCGAAAGAAAAACAGCAGAA TATTGATGCCACCTGGCAGGCGCTCTCCTCCATG ACTCAGGAACAGGCGAATACGCTGGTGATCAAC GCCGACGAAAATATTCTGCAAGGCTGGCTGGATC TGCAGCGCGTCTGGTTTGATAACCGTAACGATCC CGACATGATGAAAGCCGGGATCGCCGACTGGCA GAAACGTTATCCGAACAATCCGGGCGCGAAAAT GCTGCCAACGCAGTTGGTTAACGTAAAAGCGTTT AAACCAGCCTCGACCAACAAAATCGCCCTGCTGT TGCCACTGAATGGCCAGGCAGCGGTATTTGGTCG CACTATTCAGCAAGGCTTTGAAGCGGCGAAAAAT ATCGGCACTCAGCCAGTGGCAGCTCAGGTAGCTG CCGCACCTGCCGCAGACGTAGCTGAACAACCTCA GCCGCAAACCGTGGATGGCGTTGCCAGCCCGGCA CAAGCCTCGGTTAGCGATCTGACCGGTGAACAGC CTGCAGCCCAGCCGGTGCCTGTAAGCGCCCCGGC GACAAGCACCGCAGCGGTAAGCGCACCCGCAAA TCCATCCGCAGAGCTGAAAATCTACGATACCTCA TCACAACCACTTAGCCAGATCTTAAGCCAGGTTC AGCAGGATGGCGCGAGTATTGTGGTCGGTCCGTT GCTGAAAAATAACGTTGAAGAGTTGCTGAAGAG CAACACTCCGCTGAACGTACTGGCACTGAACCAG CCGGAGAATATCGAAAATCGCGTCAATATTTGTT |
| | | ACTTCGCGCTTTCACCGGAAGACGAAGCGCGCGA TGCAGCGCGTCATATTCGTGACCAGGGTAAACAA GCGCCGCTGGTGCTGATCCCACGCAGTTCATTGG GCGATCGCGTAGCCAATGCGTTTGCGCAAGAGTG GCAGAAACTGGGCGGCGGCACCGTTCTGCAACA AAAATTTGGTTCCACCAGCGAATTACGCGCGGGT GTTAACGGCGGTTCTGGTATTGCTTTAACGGGTA GCCCGATTACTCTCAGAGCGACAACCGACTCCGG CATGACGACCAACAATCCAACGCTGCAAACCACG CCAACCGATGACCAGTTCACCAATAATGGCGGTC GTGTCGATGCGGTGTACATTGTGGCAACGCCGGG TGAAATCGCTTTTATCAAACCGATGATCGCCATG CGTAACGGTAGCCAGAGCGGTGCAACGCTGTACG CCAGCTCCCGCAGTGCGCAAGGGACCGCTGGCCC GGATTTCCGACTGGAGATGGAAGGCTTGCAGTAC AGCGAAATCCCGATGCTGGCAGGCGGTAATCTAC CGTTAATGCAGCAGGCACTCAGCGCGGTGAATAA CGATTATTCACTGGCTCGCATGTATGCGATGGGC GTCGATGCCTGGTCGCTGGCAAATCATTTCTCAC AAATGCGCCAGGTTCAGGGTTTTGAAATCAACGG TAATACCGGAAGCCTGACGGCTAACCCGGATTGC GTGATTAACAGGAACTTATCATGGCTACAGTACC AACAAGGTCAGGTAGTCCCCGTCAGTTAA |
| 81 | Nucleotide sequence of lpoA signal | ATGGTACCCTCAACATTTTCTCGTTTGAAAGCCGC GCGTTGTCTGCCTGTTGTTCTGGCAGCCCTGATTT TCGCCGGT |
| 82 | Structural gene of lpoB | TGTGTGGGGCAACGTGAACCTGCACCGGTAGAAG AAGTGAAACCAGCGCCGGAACAACCAGCCGAGC CACAACAGCCTGTCCCCACAGTGCCCTCGGTGCC GACGATCCCGCAGCAGCCAGGCCCAATTGAGCAC GAAGATCAAACTGCACCGCCTGCGCCGCATATTC GCCATTATGACTGGAATGGCGCAATGCAGCCGAT GGTCAGTAAGATGCTTGGGGCTGACGGGGTGACT GCGGGTAGCGTCCTGCTGGTTGATAGCGTTAACA ACCGTACTAACGGTTCGCTGAATGCCGCAGAAGC GACCGAAACGCTGCGAAATGCGCTGGCTAATAAC GGGAAATTTACCCTGGTTTCCGCCCAGCAACTGT CGATGGCGAAGCAACAGTTAGGTTTGTCGCCGCA GGACAGTTTAGGCACCCGTAGTAAAGCCATAGGC ATTGCCCGCAATGTCGGCGCTCATTACGTGCTGT ACTCCAGCGCCTCTGGCAACGTTAACGCTCCGAC CCTACAAATGCAGCTGATGCTGGTGCAGACGGGC |
| | | GAAATTATCTGGTCAGGTAAAGGTGCCGTTTCGC AGCAATAA |
| 83 | Nucleotide sequence of lpoB signal | ATGACAAAAATGAGTCGCTACGCCTTGATTACCG CGCTGGCGATGTTTCTCGCCGGG |

All publications, patents, and patent applications cited herein are incorporated herein by reference in their entirety.

## Claims

1. A method for producing plasmid DNA, comprising the following steps (a) to (c):
(a) a step of preparing *E. coli,* which has a mutation in a gene region associated with maintaining outer membrane properties and has a desired plasmid;
(b) a step of culturing *E. coli* of (a); and
(c) a step of recovering a desired plasmid from bacterial cells after culture.

2. The method according to claim 1, wherein the gene region associated with maintaining outer membrane properties is a gene region associated with maintaining physical and/or mechanical outer membrane properties.

3. The method according to claim 2, wherein the gene region associated with maintaining physical and/or mechanical outer membrane properties is a gene region directly associated with maintaining physical and/or mechanical outer membrane properties.

4. The method according to claim 3, wherein the gene region directly associated with maintaining physical and/or mechanical outer membrane properties is at least one selected from the group consisting of sequences encoding structural proteins of *pal, lpp, ompA, ybiS, ycfS, erfK, tolA, tolB, nlpl, bamD, bamA, lolA, lolB, lolC, lolD, lolE, lptE, IptD, lptA, IptB, lptF, lptG, slyB, mrcA, mrcB, lpoA, lpoB, tolQ,* and *tolR*; and nucleotide sequences encoding their signal peptides, promoter sequences, and SD sequences encoding their signal peptides.

5. The method according to claim 1, wherein the *E. coli* has a mutation in an amino acid sequence of at least one peptide selected from the group consisting of: Pal, Lpp, OmpA, YbiS, YcfS, ErfK, TolA, TolB, Nlpl, BamD, BamA, LolA, LolB, LolC, LolD, LolE, LptE, LptD, LptA, LptB, LptF, LptG, SlyB, MrcA, MrcB, LpoA, LpoB, TolQ, and TolR; and signal peptides thereof.

6. The method according to claim 5, wherein the *E. coli* has a mutation in an amino acid sequence of at least one peptide selected from the group consisting of Pal, Lpp, OmpA, YbiS, YcfS, ErfK, and signal peptides thereof.

7. The method according to claim 1, wherein the mutation is a complete disruption.

8. The method according to claim 1, wherein the mutation is a partial mutation.

9. The method according to claim 8, wherein the mutation is a partial mutation of a signal peptide.

10. The method according to claim 8, wherein the mutation is a partial mutation of a structural protein.

11. The method according to claim 10, wherein the structural protein is a structural protein having a partial mutation of any of the following (A) to (E):
(A) a structural protein binding to or contacting with any of an outer membrane, a peptidoglycan layer, or an inner membrane and thus having a substitution or disruption in an amino acid residue at the site of binding or contacting and/or in the vicinity thereof;
(B) a structural protein having an enzymatic activity of allowing another protein to bind to a peptidoglycan layer and having a substitution or disruption in an amino acid residue at its active site and/or in the vicinity thereof;
(C) a structural protein serving as an enzyme involved in forming a peptidoglycan layer and having a substitution or disruption in an amino acid residue at its active site and/or in the vicinity thereof;
(D) a structural protein serving as a protein involved in outer membrane transport of a lipoprotein present in a periplasm fraction and having a substitution or deletion in an amino acid residue at a site binding to the lipoprotein and/or in the vicinity thereof; and
(E) a structural protein serving as a protein involved in outer membrane transport of a lipopolysaccharide and having a substitution or deletion in an amino acid residue at a site binding to the lipoprotein and/or in the vicinity thereof.

12. The method according to claim 11, wherein the structural protein is the structural protein (A), which is at least one selected from the group consisting of Pal, Lpp, and OmpA.

13. The method according to claim 12, wherein the structural protein is Pal, which has the mutation in an N-terminal region thereof.

14. The method according to claim 12, wherein the structural protein is Lpp, which has the mutation in a C-terminal region thereof.

15. The method according to claim 12, wherein the structural protein is OmpA, which has the mutation in a C-terminal region thereof.

16. The method according to claim 11, wherein the structural protein is the structural protein (B), which is at least one selected from the group consisting of YbiS, YcfS, and ErfK.

17. The method according to claim 1, wherein the *E. coli* is derived from the B strain or the K12 strain.

18. A method for preparing *E. coli* for plasmid DNA production, comprising the steps (i) and (ii):
(i) a step of causing a mutation in a gene region associated with maintaining outer membrane properties of *E. coli*; and
(ii) a step of preparing *E. coli,* which has the mutation obtained in (i) and has a desired plasmid.

19. *E. coli* for producing plasmid DNA, which has a mutation in a gene region associated with maintaining outer membrane properties and has a desired plasmid.
